# EUROPEAN PATENT APPLICATION

(11) **EP 4 043 012 A1**
(43) Date of publication of application: **17.08.2022**
(21) Application number: 20872584.6
(22) Date of filing: 29.09.2020
(51) Int. Cl.: A61K 31/216, A61K 31/192, A61K 31/085, A61K 31/352, A61K 31/621, A61K 31/37, A61K 31/05, A61K 31/12, A61P 9/00

(54) **DRUG FOR TREATING ARTERY-RELATED DISEASES, AND USE THEREOF**

(30) Priority: 30.09.2019 CN 201910943781
(71) Applicant: Shanghai Institute of Materia Medica, Chinese Academy of Sciences, Pudong, Shanghai 201203 (CN)
(72) Inventor: JIANG, Baohong, Shanghai 201203 (CN); ZHANG, Xianjing, Shanghai 201203 (CN); DING, Yuchao, Shanghai 201203 (CN); ZHAI, Ziyi, Shanghai 201203 (CN)
(74) Representative: Cabinet Laurent & Charras
(86) International application number: PCT/CN2020/118945
(87) International publication number: WO 2021/063366

(57) **Abstract**

Provided are a drug for treating artery-related diseases and the use thereof. Specifically, provided are the use of a class of compounds of formula I in the treatment of artery-related diseases. Experiments show that the compounds of formula I have a significant effect on aneurysm, intramural hematoma and/or atrerial dissection.

## Description

### TECHNICAL FIELD

The present invention relates to the field of medicine and particularly to a drug for treating artery-related diseases and use thereof.

### BACKGROUND

Aneurysms, intramural hematomas, and arterial dissections are interrelated and independent diseases, which can occur in thoracic aorta, abdominal aorta, splenic artery, hepatic artery, superior mesenteric artery, coeliac axis artery, renal artery, epiploon artery, inferior mesenteric artery, intracranial artery, carotid artery and the like. All these diseases may result in vascular rupture when they develop into advanced stage, and endanger the patient's life. The causes of aneurysms, intramural hematomas, and arterial dissections are very complex. The most common risk factors comprise smoking, hypertension and atherosclerosis. It is characterized as degradation of extracellular matrix inmedia and adventitia, arterial wall thinning, and invasion of inflammatory cells. Vascular rupture is the main cause of patient death as the disease progresses.

Clinically, there are no preventive and therapeutic drugs for aneurysms, intramural hematomas, and arterial dissections. Except for surgical treatment, there is no drug for treating these patients.

Therefore, there is an urgent need for drugs in clinic that are useful to treat aneurysms, intramural hematomas, and/or arterial dissections, thereby giving patients more therapeutic options.

### SUMMARY OF THE INVENTION

An object of this invention is to provide a use of a compound of formula I for manufacturing a drug used for treatment of aneurysms, intramural hematoma and/or arterial dissections.

In the first aspect of the present invention, it provides a use of a compound of formula I, or an isomer, a crystal form, a hydrate or a solvate thereof, or a pharmaceutically acceptable salt thereof, for manufacturing a pharmaceutical composition or formulation; wherein the pharmaceutical composition or formulation is used for prevention and/or treatment of an arteriopathy selected from: (i) aneurysm; (ii) intramural hematoma; and/or (iii) arterial dissection; 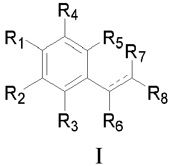
wherein, R₁, R₂, R₃, R₄ and R₅ are each independently selected from the group consisting of H, halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
R₆ and R₇ are each independently selected from the group consisting of H, oxo (=O), halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
each R₁₀ is independently selected from the group consisting of -(C=O)-substituted or unsubstituted C1-C8 alkyl, -(C=O)-substituted or unsubstituted C6-C10 aryl, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₇ together with an atom to which they are attached form substituted or unsubstituted C3-C8 cycloalkyl, and substituted or unsubstituted 3-8 membered heterocycloalkyl having 1-3 heteroatoms selected from N, S and O;
" 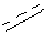 " is a double bond or a single bond;
R₅ is selected from the group consisting of H, -(C=O)-R₉, substituted or unsubstituted C1-C8 alkyl, substituted or unsubstituted C2-C8 alkenyl, substituted or unsubstituted C2-C8 alkynyl, and =CRₐR_{b};
R₉ is selected from the group consisting of H, hydroxy, substituted or unsubstituted C1-C8 alkyl, substituted or unsubstituted C2-C8 alkenyl, substituted or unsubstituted C2-C8 alkynyl, -NRₐR_{b}, and -O-R₁₁;
R₁₁ is selected from the group consisting of substituted or unsubstituted C1-C8 alkyl, substituted or unsubstituted C2-C8 alkenyl, substituted or unsubstituted C2-C8 alkynyl, -(substituted or unsubstituted C1-C8 alkylene)-C6-C10 aryl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₉ form 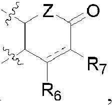 wherein Z is O, NRₐ or S;
unless especially indicated, the "substituted" refers to be substituted with one or more (e.g., 2, 3 or 4) substituents selected from the group consisting of halogen, deuterium, C1-C6 alkyl, C2-C6 alkenyl, C2-C6 alkynyl, C1-C6 alkoxy, halogenated C1-C6 alkyl, halogenated C1-C6 alkoxy, oxo (=O), -CN, -OH, -N(Rₐ)R_{b}, carboxyl, C1-C6 ester group (-C(=O)-OC1-C5 alkyl or -O-C(=O)C1-C5 alkyl), or substituted or unsubstituted group selected from the group consisting of: C1-C6 alkyl, C3-C8 cycloalkyl, C1-C6 amine group, C1-C6 ester group, C6-C10 aryl, 5-10 membered heteroaryl having 1-3 heteroatoms selected from N, S and O, 5-10 membered heterocyclyl having 1-3 heteroatoms selected from N, S and O, -(CH₂)-C6-C10 aryl, -(CH₂)-(5-10 membered heteroaryl having 1-3 heteroatoms selected from N, S and O), and the substituent is selected from the group consisting of: halogen, C1-C6 alkyl, C1-C6 alkynyl, C1-C6 alkoxy, oxo, -CN, -NH₂, -OH, C6-C10 aryl, C1-C6 amine group, C2-C6 amide group, and 5-10 heteroaryl having 1-3 heteroatoms selected from N, S and O;
each of Rₐ and R_{b} is independently selected from H, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O.

In another preferred embodiment, R₁, R₂, R₃, R₄, R₅, R₆ and R₇ are each independently selected from the group consisting of H, halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
R₁₀ is selected from the group consisting of -(C=O)-substituted or unsubstituted C1-C8 alkyl, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₇ together with an atom to which they are attached form substituted or unsubstituted C3-C8 cycloalkyl, or substituted or unsubstituted 3-8 membered heterocycloalkyl having 1-3 heteroatoms selected from N, S and O;
" 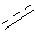 " is a double bond or a single bond;
Rs is selected from the group consisting of -(C=O)-R₉, substituted or unsubstituted C1-C8 alkyl, substituted or unsubstituted C2-C8 alkenyl, substituted or unsubstituted C2-C8 alkynyl, and =CRₐR_{b};
R₉ is selected from the group consisting of H, hydroxy, substituted or unsubstituted C1-C8 alkyl, substituted or unsubstituted C2-C8 alkenyl, substituted or unsubstituted C2-C8 alkynyl, -NRₐR_{b}, and -O-R₁₁;
R₁₁ is selected from the group consisting of substituted or unsubstituted C1-C8 alkyl, substituted or unsubstituted C2-C8 alkenyl, substituted or unsubstituted C2-C8 alkynyl, -substituted or unsubstituted C1-C8 alkylene-C6-C10 aryl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or Rs and R₉ form 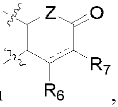 wherein Z is O, NRₐ or S;
unless especially indicated, the "substituted" refers to be substituted with one or more (e.g., 2, 3 or 4) substituents selected from the group consisting of halogen, deuterium, C1-C6 alkyl, C2-C6 alkenyl, C2-C6 alkynyl, C1-C6 alkoxy, halogenated C1-C6 alkyl, halogenated C1-C6 alkoxy, oxo (=O), -CN, -OH, -N(Rₐ) R_{b}, carboxyl, C1-C6 ester group (-C(=O)-OC1-C5 alkyl or -O-C(=O)C1-C5 alkyl), or substituted or unsubstituted group selected from the group consisting of: C1-C6 alkyl, C3-C8 cycloalkyl, C1-C6 amine group, C1-C6 ester group, C6-C10 aryl, 5-10 membered heteroaryl having 1-3 heteroatoms selected from N, S and O, 5-10 membered heterocyclyl having 1-3 heteroatoms selected from N, S and O, -(CH₂)-C6-C10 aryl, -(CH₂)-(5-10 membered heteroaryl having 1-3 heteroatoms selected from N, S and O), and the substituent is selected from the group consisting of: halogen, C1-C6 alkyl, C1-C6 alkynyl, C1-C6 alkoxy, oxo, -CN, -NH₂, -OH, C6-C10 aryl, C1-C6 amine group, C2-C6 amide group, and 5-10 heteroaryl having 1-3 heteroatoms selected from N, S and O;
each of Rₐ and R_{b} is independently selected from H, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O.

In another preferred embodiment, each of R₁, R₂, R₃, R₄, R₅, R₆, R₇ and R₈ independently comprises the corresponding group of each compound in Table. 1.

In another preferred embodiment, " 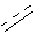 " is a double bond.

In another preferred embodiment, " 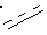 " is a single bond and R₇ is hydroxy. In another preferred embodiment, at least one (1, 2 or 3) of R₁, R₂, R₃, R₄ and R₅ is hydroxy or C1-C8 alkoxy.

In another preferred embodiment, R₁ is hydroxy, C1-C3 alkoxy or C1-C3 alkyl.

In another preferred embodiment, R₂ is hydroxy, C1-C3 alkoxy or C1-C3 alkyl.

In another preferred embodiment, at least one of R₁ and R₂ is hydroxy or C1-C8 alkoxy.

In another preferred embodiment, R₆ is oxo and R₇ is H.

In another preferred embodiment, both R₆ and R₇ cannot be oxo (=O) at the same time.

In another preferred embodiment, the compound of formula I has a structure of Ia: 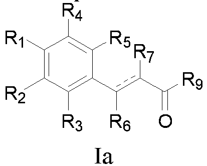 wherein, R₁, R₂, R₃, R₄, R₅, R₆, R₇, R₉ and " 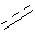 " are as defined hereinabove.

In another preferred embodiment, R₉ is hydroxy or unsubstituted or substituted C1-C8 alkoxy-C6-C10 aryl.

In another preferred embodiment, both R₁ and R₉ are OH.

In another preferred embodiment, R₁ and R₂ are each independently hydroxy or C1-C8 alkoxy, " 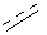 " is double bond and R₉ is hydroxy or substituted or unsubstituted C1-C8 alkoxy.

In another preferred embodiment, the "substituted C1-C8 alkoxy-C6-C10 aryl" includes -C2-C8 ester substituted ethylene-(substituted or unsubstituted phenyl), wherein a substituent on the phenyl is -OH, or -OCH₃.

In another preferred embodiment, the compound has a structure of Ib: 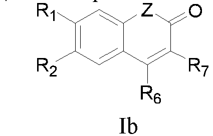 wherein, R₁, R₂, R₆, R₇ and Z are as defined hereinabove.

In another preferred embodiment, the compound has a structure of Ic: 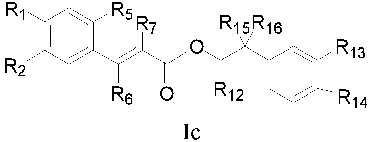 wherein, R₁, R₂, R₅, R₆ and R₇ are as defined hereinabove,
R₁₂ is selected from the group consisting of: H, halogen, -OH, carboxyl, -C2-C8 ester group, substituted or unsubstituted C1-C6 alkyl, and substituted or unsubstituted C1-C6 alkoxy;
R₁₃ and R₁₄ are each independently selected from the group consisting of H, -OH, halogen, C1-C4 alkyl, C1-C4 haloalkyl, and C1-C6 alkoxy;
R₁₅ and R₁₆ are each independently selected from H, halogen, -OH, carboxyl, substituted or unsubstituted C1-C6 alkyl, substituted or unsubstituted C1-C6 alkoxy.

In another preferred embodiment, R₁₂ is -(C=O)-O-C1-C6 alkyl.

In another preferred embodiment, R₁₃ and R₁₄ are each independently hydroxy or C1-C3 alkoxy.

In another preferred embodiment, R₁₅ and R₁₆ are each independently hydrogen.

In another preferred embodiment, the compound is selected from the following group A:

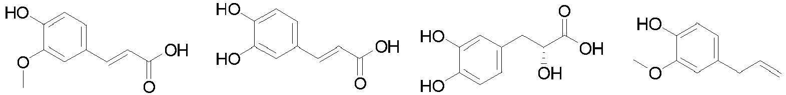

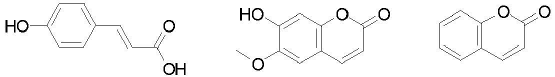

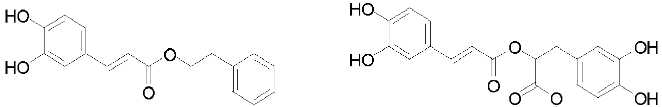

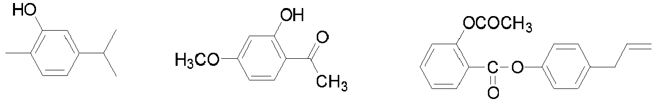

In another preferred embodiment, the compound is selected from the group consisting of ferulic acid, caffeic acid, danshensu, eugenol, coumarin, p-coumarin, scopoletin, phenethyl caffeate and methyl rosmarinate.

In another preferred embodiment, the pharmaceutically acceptable salt comprises piperazine ferulate and sodium ferulate.

In another preferred embodiment, the pharmaceutically acceptable ester comprises methyl rosmarinate and aspirin eugenol ester.

In another preferred embodiment, the pharmaceutical composition further comprises a pharmaceutically acceptable carrier.

In another preferred embodiment, the artery is selected from the group consisting of thoracic aorta, abdominal aorta, splenic artery, hepatic artery, superior mesenteric artery, coeliac axis artery, renal artery, epiploon artery, inferior mesenteric artery, intracranial artery, carotid artery and a combination thereof.

In another preferred embodiment, the aneurysm is selected from the group consisting of early aneurysm, mid-term aneurysm, late aneurysm and a combination thereof.

In another preferred embodiment, the dosage form of the pharmaceutical composition is selected from the group consisting of liquid formulation (e.g., solution, emulsion, suspension) and solid formulation (e.g., a lyophilized formulation).

In another preferred embodiment, the dosage form of the pharmaceutical composition is selected from the group consisting of injection (e.g., injection solution or powder), oral formulation (e.g., capsule, tablet, pill, powder, granule, syrup, oral liquid or tincture), and preferably, the dosage form is oral formulation.

In the second aspect of the present invention, it provides a use of a medicinal material or food material containing a compound of formula I and/or an extract containing the compound of formula I, for manufacturing a composition; wherein the composition is used for prevention and/or treatment of an arteriopathy selected from: (i) aneurysm; (ii) intramural hematoma; and/or (iii) arterial dissection; 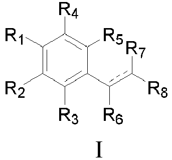 wherein, R₁, R₂, R₃, R₄, R₅, R₆, R₇ and " 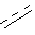 " are as defined hereinabove;
and, the compound of formula I is selected from the group consisting of: ferulic acid, caffeic acid, Danshensu (or salvianic acid A), phenethyl caffeinate, methyl rosmarinate, coumarin, *p*-coumarin acid, scopoletin, eugenol, carvacrol, paeonol, aspirin eugenol ester, and a combination thereof.

In another preferred embodiment, the medicinal material comprises Chinese herbal medicines.

In another preferred embodiment, the medicinal material is selected from: propolis *(Colla Apis),* peppermint *(Mentha haplocalyx Briq.), Ferulae Resina, Angelicae Sinensis Radix, Selaginellae Herba, Equiseti Hiemalis Herba, Chuanxiong Rhizoma, Cimicifugae Rhizoma, Ziziphi Spinosae Semen, Flos Anisopappi Chinensis, Caryophylli Flos, Stachys Palustris L., Fagopyri Dibotryis Rhizoma, Elaeagnus Multiflora Thunb, Cnidii Fructus, Rabdosiae Rubescentis Herba, Vitex Negundo L., South Salviae Miltiorrhizae Radix Et Rhizoma, Rabdosia Serra, Fraxini Cortex, Artemisiae Scopariae Herba, Angelicae Pubescentis Radix, Daphne odora Thunb. Hedyotis diffusa, Ginseng Radix Et Rhizoma, Cuscutae Semen, leaf of Juglans regia L., Murrayae Folium Et Cacumen, Cinnamomum Purpureum, Alpinia Galanga (L.) Willd, Magnoliae Flos, Narcissus Tazetta L., Commiphora Myrrha Engl., Rosae Rugosae Flos, Thymus Mongolicus Ronn, Cestrum Nocturnum L.,* and a combination thereof.

In another preferred embodiment, the food material is selected from the group consisting of: coffea, chaff, vanilla bean, wheat bran, rice bran, peppermint, *Perillae Folium,* buckwheat, *Artemisia ounghusbandii,* onion, and a combination thereof.

In another preferred embodiment, the extract comprises an extract extracted from a substance selected from the group consisting of propolis, peppermint, *Ferulae Resina, Angelicae Sinensis Radix, Selaginellae Herba, Equiseti Hiemalis Herba, Chuanxiong Rhizoma, Cimicifugae Rhizoma, Ziziphi Spinosae Semen, Flos Anisopappi Chinensis, Caryophylli Flos, Stachys Palustris L., Fagopyri Dibotryis Rhizoma, Elaeagnus Multiflora Thunb, Cnidii Fructus, Rabdosiae Rubescentis Herba, Vitex Negundo L., South Salviae Miltiorrhizae Radix Et Rhizoma, Rabdosia Serra, Fraxini Cortex, Artemisiae Scopariae Herba, Angelicae Pubescentis Radix, Daphne odora Thunb. Hedyotis diffusa, Ginseng Radix Et Rhizoma, Cuscutae Semen, leaf of Juglans regia L., Murrayae Folium Et Cacumen, Cinnamomum Purpureum, Alpinia Galanga (L.) Willd, Magnoliae Flos, Narcissus Tazetta L., Commiphora Myrrha Engl., Rosae Rugosae Flos, Thymus Mongolicus Ronn, Cestrum Nocturnum L*., coffea, chaff, vanilla bean, wheat bran, rice bran, peppermint, *Perillae Folium,* buckwheat, *Artemisia ounghusbandii,* onion, and a combination thereof.

In another preferred embodiment, the composition includes a pharmaceutical composition, a food composition, a dietary supplement, or a healthcare composition.

In another preferred embodiment, the pharmaceutical composition further comprises a pharmaceutically acceptable carrier.

In the third aspect of the invention, it provides a pharmaceutical composition comprising:
(a) a first active ingredient selected from the group consisting of: a compound of formula I, an isomer thereof, a crystal form thereof, a hydrate or solvate thereof, or a pharmaceutically acceptable salt thereof, and a combination thereof; the compound of formula I is as described in claim 1;
(b) a second active ingredient selected from the group consisting of: polymeric salvianolic acid, a stereoisomer thereof, a crystal form thereof, a pharmaceutically acceptable salt thereof, and a combination thereof; and
(c) a pharmaceutically acceptable carrier.

In another preferred embodiment, the compound is selected from the group consisting of ferulic acid, caffeic acid, Danshensu, phenethyl caffeinate, methyl rosmarinate, coumarin, *p*-coumarin, eugenol, scopoletin, and a combination thereof.

In another preferred example, the polymeric salvianolic acid is selected from the group consisting of rosmarinic acid, salvianolic acid C, violic acid or salvianolic acid A, salvianolic acid B, and a combination thereof.

In another preferred embodiment, the compound of formula I is ferulic acid, and the polymeric salvianolic acid is rosmarinic acid.

In the fourth aspect of the invention, it provides a method of preventing and/or treating (i) aneurysm; (ii) arterial intramural hematoma; and/or (iii) arterial dissection, which comprises a step of: administering a therapeutically effective amount of the compound of formula I, an isomer thereof, a crystal form thereof, a hydrate or a solvate thereof, or a pharmaceutically acceptable salt thereof, or a pharmaceutical composition of the present invention to a subject in need.

In another preferred embodiment, the subject is a mammal.

In another preferred embodiment, the subject is human, mouse or rat.

It should be understood that within the scope of the present invention, each technical features of the present invention described above and in the following (as examples) may be combined with each other to form a new or preferred technical solution, which is not redundantly repeated one by one here due to space limitation.

### DESCRIPTION OF FIGURE

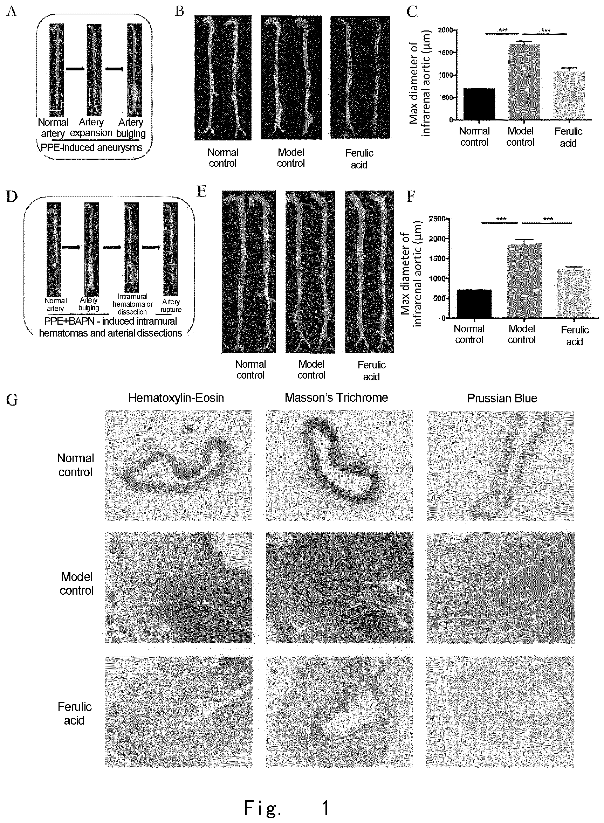

Figure 1 shows the experimental results of ferulic acid inhibiting aneurysms, intermural hematomas, and/or arterial dissections. A is the schematic diagram of the aneurysm model-making method; B is the general view of the aneurysm expansion in each group; C is the quantification of the maximum diameter of the aneurysms; D is the schematic diagram of the model-making method of aneurysm, intramural hematoma and arterial dissection; E is the general view of each arteries of aneurysm, intramural hematoma and arterial dissection models in each group; F is the quantification of the maximum blood vessel diameter of arteriopathy sites (aneurysms, intramural hematomas or dissection ruptures); and G is the histological evaluation with hematoxylin-eosin (HE) staining, Masson staining and Prussian blue staining of aneurysm, intersquash hematoma and arterial dissection models in each group.

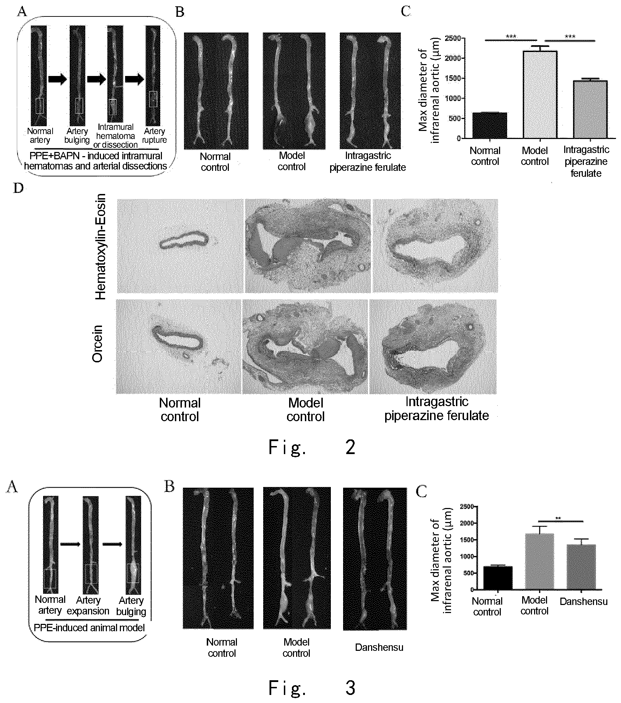

Figure 2 shows the experimental results of piperazine ferulate (intragastric administration) inhibiting aneurysms, intermural hematomas, and/or arterial dissections. A is the schematic diagram of the model-making method of aneurysm, intramural hematoma and arterial dissection; B is the general view of arteries in each group; C is the quantification of the maximum blood vessel diameter of arteriopathy sites; and D is the histological evaluation with hematoxylin-eosin staining and orcein staining in each group.
Figure 3 shows the experimental results of Danshensu inhibiting aneurysms. A is the schematic diagram of the model-making method of aneurysm; B is the general view of the aneurysm expansion in each group; and C is the quantification of the maximum diameter of the aneurysms.

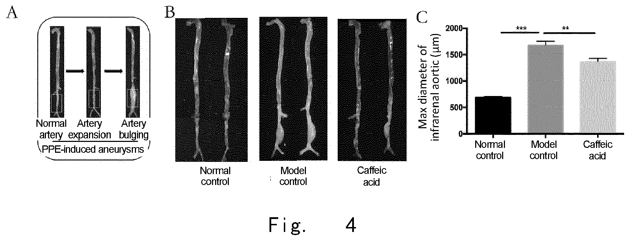

Figure 4 shows the experimental results of caffeic acid inhibiting aneurysms. A is the schematic diagram of the model-making method of aneurysm; B is the general view of the aneurysm expansion in each group; C is the quantification of the maximum diameter of the aneurysms.

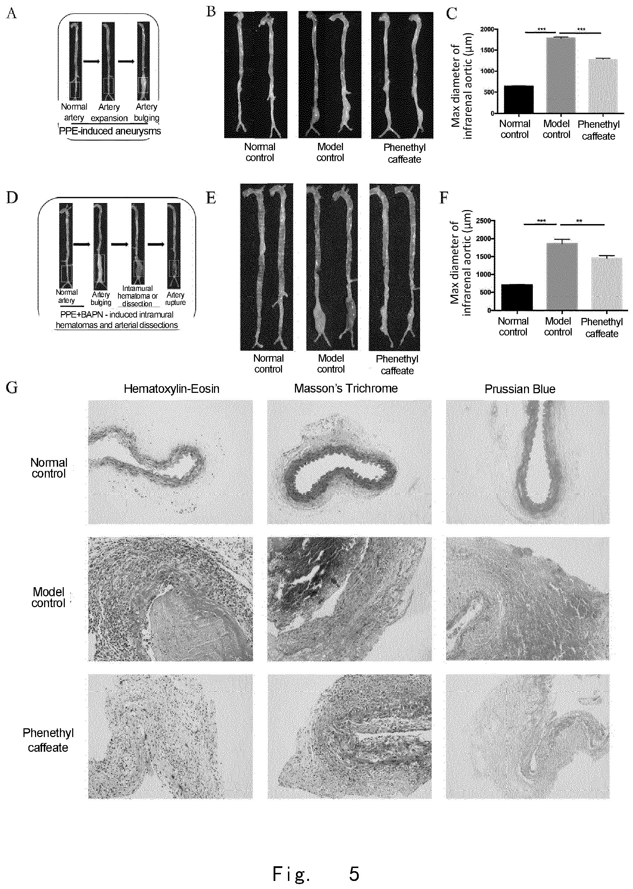

Figure 5 shows the experimental results of phenethyl caffeate inhibiting aneurysms, intermural hematomas, and/or arterial dissections. A is the schematic diagram of the aneurysm model-making method; B is the general view of the aneurysm expansion in each group; C is the quantification of the maximum diameter of the aneurysms; D is the schematic diagram of the model-making method of aneurysm, intramural hematoma and arterial dissection; E is the general view of each arteries of aneurysm, intramural hematoma and arterial dissection models in each group; F is the quantification of the maximum blood vessel diameter of arteriopathy sites; and G is the histological evaluation with hematoxylin-eosin staining, Masson staining and Prussian blue staining of aneurysm, intersquash hematoma and arterial dissection models in each group.

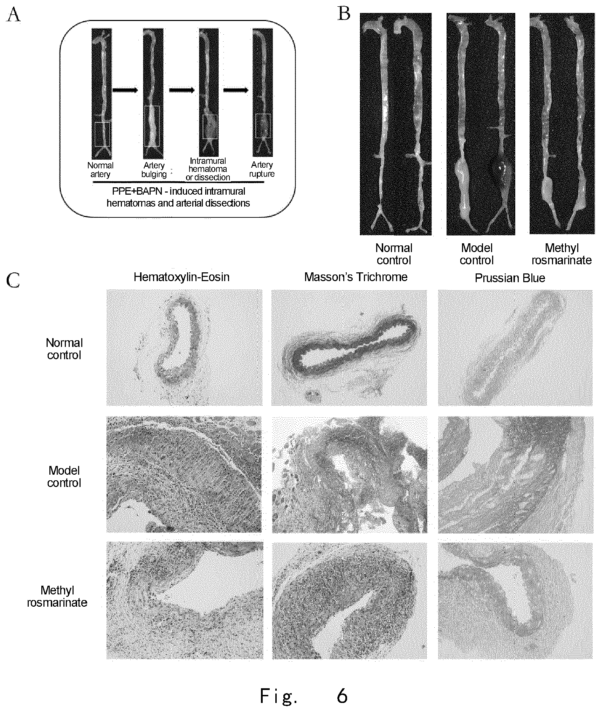

Figure 6 shows the experimental results of methyl rosmarinate inhibiting aneurysms, intermural hematomas, and arterial dissections. A is the schematic diagram of the model-making method of aneurysm, intramural hematoma and arterial dissection; B is the general view of arteries in each group; and C is the histological evaluation with hematoxylin-eosin staining, Masson staining and Prussian blue staining in each group.

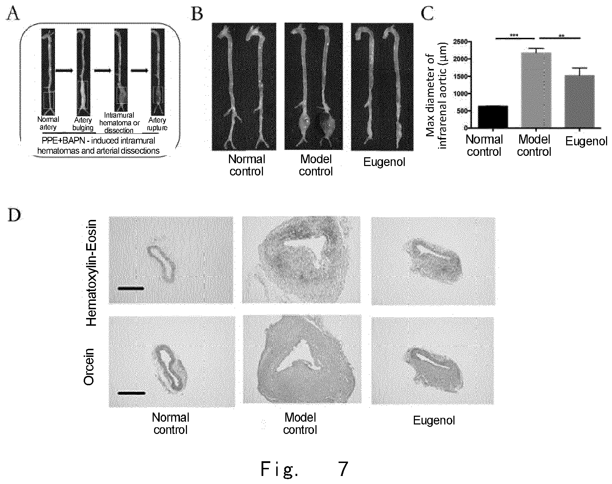

Figure 7 shows the experimental results of intraperitoneal injected eugenol inhibiting aneurysms, intermural hematomas, and arterial dissections. A is the schematic diagram of the model-making method of aneurysm, intramural hematoma and arterial dissection; B is the general view of arteries in each group; C is the quantification of the maximum blood vessel diameter of arteriopathy sites; and D is the histological evaluation with hematoxylin-eosin staining and orcein staining in each group.

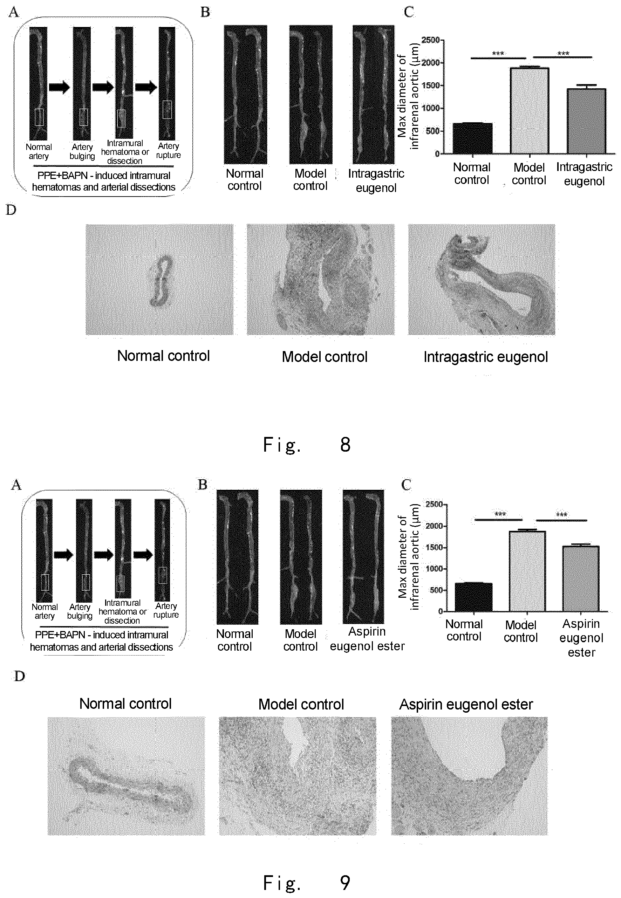

Figure 8 shows the experimental results of oral intragastric administered eugenol inhibiting aneurysms, intermural hematomas, and arterial dissections; A is the schematic diagram of the model-making method of aneurysm, intramural hematoma and arterial dissection; B is the general view of arteries in each group; C is the quantification of the maximum blood vessel diameter in each group; and D is the histological evaluation with hematoxylin-eosin staining in each group.
Figure 9 shows the experimental results of aspirin eugenol ester inhibiting aneurysms, intermural hematomas, and arterial dissections; A is the schematic diagram of the model-making method of aneurysm, intramural hematoma and arterial dissection; B is the general view of arteries in each group; C is the quantification of the maximum blood vessel diameter in each group; and D is the histological evaluation with hematoxylin-eosin staining in each group.

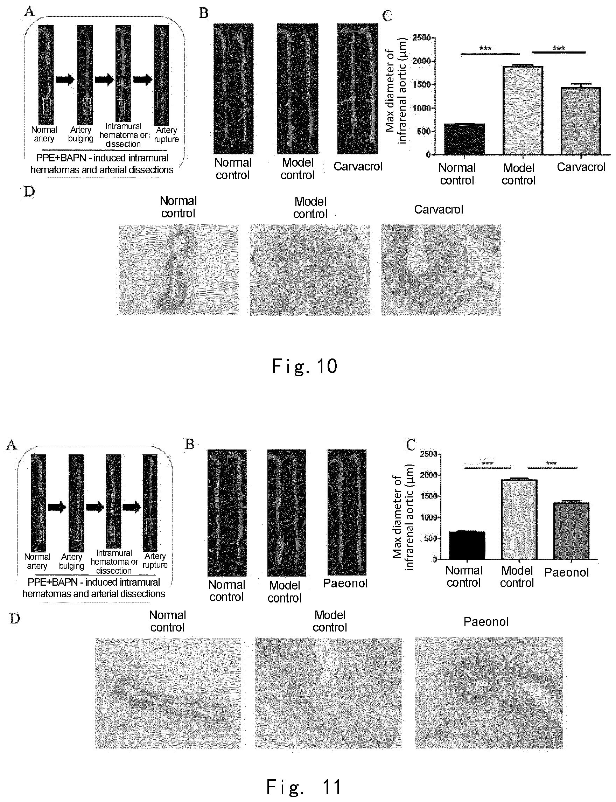

Figure 10 shows the experimental results of carvacrol inhibiting aneurysms, intermural hematomas, and arterial dissections. A is the schematic diagram of the model-making method of aneurysm, intramural hematoma and arterial dissection; B is the general view of the aneurysm expansion in each group; C is the quantification of the maximum blood vessel diameter in each group; and D is the histological evaluation with hematoxylin-eosin staining.
Figure 11 shows the experimental results of paeonol inhibiting aneurysms, intermural hematomas, and arterial dissections. A is the schematic diagram of the model-making method of aneurysm, intramural hematoma and arterial dissection; B is the general view of the aneurysm expansion in each group; C is the quantification of the maximum blood vessel diameter in each group; and D is the histological evaluation with hematoxylin-eosin staining.

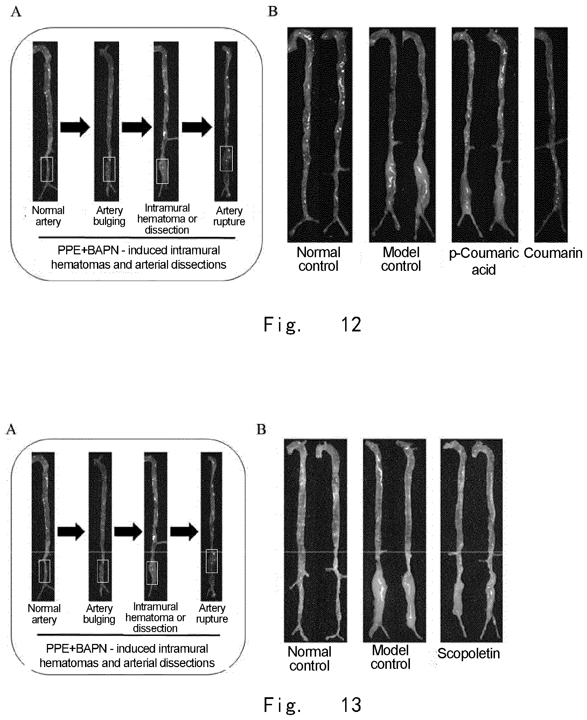

Figure 12 shows the experimental results of coumarin and *p*-coumaric acid inhibiting aneurysms; A is the schematic diagram of the model-making method of aneurysm, intramural hematoma and arterial dissection; and B is the general view of the aneurysm expansion in each group.
Figure 13 shows the experimental results of scopoletin inhibiting aneurysms; A is the schematic diagram of the model-making method of aneurysm; and B is the general view of the aneurysm expansion in each group.

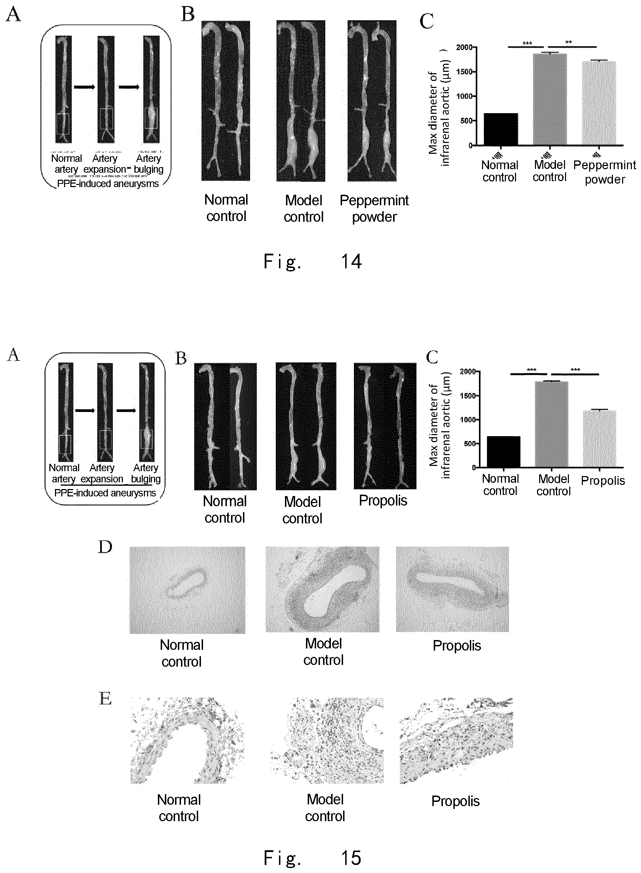

Figure 14 shows the experimental results of peppermint powder inhibiting aneurysms in the Examples; A is the schematic diagram of the model-making method of aneurysm; B is the general view of the aneurysm expansion in each group; and C is the quantification of the maximum diameter of the aneurysms.
Figure 15 shows the experimental results of propolis inhibiting aneurysms in the Examples. A is the schematic diagram of the model-making method of aneurysm; B is the general view of the aneurysm expansion in each group; C is the quantification of the maximum diameter of the aneurysms; D is the histological evaluation with hematoxylin-eosin staining; and E is the immunohistochemical results of CD68 for evaluation of macrophage infiltration.

### EMBODIMENTS

After extensive and intensive study and through a large number of screening and testing, the inventors have discovered for the first time that the compound of formula I has significant effect on treating aneurysm, intramural hematomas and arterial dissections. Surprisingly, it can not only alleviate the expansion of early and mid-term aneurysms, but also has obvious effect on treating late aneurysms, can reduce the rupture risk of late aneurysms, and can be used for aneurysms in all phases. On this basis, the present invention was completed.

### Terms

Unless otherwise defined, all technical and scientific terms used herein have the same meaning as commonly understood by those skilled in the art.

As used herein, the term "alkyl" itself or as part of another substituent, refers to a straight or branched hydrocarbon group having a specified number of carbon atoms (e.g., C1-C8, C1-C6 or C1-C3, wherein C1-C8 represents 1-8 carbon atoms). Examples of alkyl include methyl, ethyl, n-propyl, isopropyl, n-butyl, tert-butyl, isobutyl, sec-butyl, n-pentyl, n-hexyl, n-heptyl, n-octyl and the like.

As used herein, the term "alkenyl" includes straight or branched alkenyl groups. For example, C2-C8 alkenyl refers to straight or branched alkenyl groups having 2-8 carbon atoms, such as C2-C6 alkenyl or C2-C4 alkenyl, and specifically, such as vinyl, allyl, 1-propenyl, isopropenyl, 1-butenyl, 2-butenyl, and the like.

As used herein, the term "alkynyl" includes straight or branched alkynyl groups. For example, C2-C8 alkynyl refers to straight or branched alkynyl groups having 2-8 carbon atoms, such as C2-C6 alkynyl or C2-C4 alkynyl, and specifically, such as ethynyl, propynyl, butynyl, and the like.

As used herein, the term "C3-C10 cycloalkyl" refers to cycloalkyl group having 3 to 10 carbon atoms. It may be a monocyclic ring, such as C4-C7 cycloalkyl or C5-C6 cycloalkyl, specifically, such as cyclopropyl, cyclobutyl, cyclopentyl, cyclohexyl, and the like. It may also be of bicyclic form, such as bridged or spiro ring form.

As used herein, the term "C1-C8 alkoxy" refers to straight or branched alkoxy groups having 1-8 carbon atoms; such as methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O, such as C4-C7 heterocycloalkyl or C5-C6 heterocycloalkyl. It may be a monocyclic ring or bicyclic form, such as bridged or spiro ring form. Specific examples may be oxetane, azetidine, tetrahydro-2H-pyranyl, piperidinyl, tetrahydrofuranyl, morpholinyl and pyrrolidinyl, and the like.

As used herein, the term "C6-C10 aryl" refers to an aryl group having 6 to 10 carbon atoms, such as phenyl, naphthyl, and the like.

As used herein, the term "5-10 membered heteroaryl having 1-3 heteroatoms selected from the group consisting of N, S and O" refers to cyclic aromatic groups having 5-10 atoms, of which 1-3 is selected from the group consisting of N, S and O. It may be a monocyclic ring or fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl and the like.

Unless especially indicated, all the groups of the present invention include "substituted or unsubstituted" groups. Typically, all the groups of the present invention can be substituted with substituents selected from the group consisting of halogen, deuterium, C1-C6 alkyl, C2-C6 alkenyl, C2-C6 alkynyl, C1-C6 alkoxy, halogenated C1-C6 alkyl, halogenated C1-C6 alkoxy, oxo (=O), -CN, -OH, -N(Rₐ) R_{b}, carboxyl, C1-C6 ester group (-C(=O)-OC1-C5 alkyl or -O-C(=O)C1-C5 alkyl), or substituted or unsubstituted group selected from the group consisting of: C1-C6 alkyl, C3-C8 cycloalkyl, C1-C6 amine group, C1-C6 ester group, C6-C10 aryl, 5-10 membered heteroaryl having 1-3 heteroatoms selected from N, S and O, 5-10 membered heterocyclyl having 1-3 heteroatoms selected from N, S and O, -(CH₂)-C6-C10 aryl, -(CH₂)-(5-10 membered heteroaryl having 1-3 heteroatoms selected from N, S and O), and the substituent is selected from the following group: halogen, C1-C6 alkyl, C1-C6 alkynyl, C1-C6 alkoxy, oxo, -CN, -NH₂, -OH, C6-C10 aryl, C1-C6 amine group, C2-C6 amide group, 5-10 heteroaryl having 1-3 heteroatoms selected from N, S and O.

Each of Rₐ and R_{b} is independently selected from H, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, or substituted or unsubstituted 5-10 membered heteroaryl having 1-3 heteroatoms selected from N, S and O.

As used herein, "halogen" or "halogen atom" refers to F, Cl, Br, and I. More preferably, the halogen or halogen atom is selected from F, Cl or Br. "Halogenated" means substitution by an atom selected from the group consisting of F, Cl, Br, and I.

" 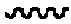 " represents the point attachment to other atoms.

As used herein, the term "isomer" is intended to include all isomeric forms (e. g., enantiomeric, diastereomeric, and geometric (or conformational isomers): for example, R, S configurations of those with asymmetric centers, (Z), (E) isomers of those containing double bonds, etc. Thus, a single stereochemical isomer of the compound of the present invention or a mixture of enantiomers, diastereomers or geometric isomers (or conformational isomers) thereof are within the scope of the present invention.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

The compound of the present invention may also contain unnatural proportion of atomic isotopes at one or more of the atoms that constitute such compounds. For example, the compounds of the present invention may be radiolabeled with radioactive isotopes, such as tritium (³H), iodine-125 (¹²⁵I), or carbon-14 (¹⁴C). All isotopic variations of the compounds of the present invention, whether radioactive or not, are encompassed within the scope of the present invention. For example, the compound can be prepared that any number of hydrogen atoms are replaced by deuterium (²H) isotope. The compound of the present invention may also contain unnatural proportion of atomic isotopes at one or more of the atoms that constitute such compounds. Unnatural proportion of an isotope may be defined as ranging from the amount found in nature to an amount consisting of 100% of the atom of interest. For example, the compounds may incorporate radioactive isotopes, such as for example tritium (³H), iodine-125 (¹²⁵I) or carbon-14 (¹⁴C), or non-radioactive isotopes, such as deuterium (²H) or carbon-13 (¹³C).

As used herein, the term "treat" or "treatment" includes disease regulating treatment and symptomatic treatment, either of which can be preventive (i.e., before t onset of symptoms, to prevent, delay or reduce the severity of symptoms)) or therapeutic (i.e., after onset of symptoms, to reduce the severity and/or duration of symptoms ).

### Active Ingredient

As used herein, "compound of the invention" refers to the compound of formula I and the isomer, crystal form, pharmaceutically acceptable salt or ester, hydrate or solvate thereof.

As used herein, "pharmaceutically acceptable salt" refers to a salt suitable for use as a medicament formed by the compound of the present invention and an acid or base. Pharmaceutically acceptable salts include inorganic and organic salts. A class of preferred salts is salts formed by the compounds of the present invention and an acid. Acids suitable for forming the salt include, but are not limited to, inorganic acids such as hydrochloric acid, hydrobromic acid, hydrofluoric acid, sulfuric acid, nitric acid, phosphoric acid, etc., organic acid such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulfonic acid, benzenesulfonic acid, etc.; and acidic amino acids such as aspartic acid and glutamic acid, etc. A class of preferred salt is the salt formed by the compounds of the present invention and a base. Bases suitable for froming the salt include, but are not limited to, inorganic bases such as sodium hydroxide, potassium hydroxide, sodium carbonate, sodium bicarbonate, sodium phosphate, etc., organic bases such as ammonia, triethylamine, diethylamine, piperazidine, etc.

As used herein, "pharmaceutically acceptable ester" refers to an ester suitable for use as a drug formed by the active ingredient compound of the present invention and an acid or alcohol. A class of preferred ester is esters formed by one or more hydroxyl groups of the active ingredient of the present invention with an acid, An acid suitable for forming the ester includes, but is not limited to, phosphoric acid, formic acid, acetic acid, propionic acid, oxalic acid, malonic acid, succinic acid, fumaric acid, maleic acid, lactic acid, malic acid, tartaric acid, citric acid, picric acid, methanesulfonic acid, benzanesulfonic acid, benzenesulfonic acid, etc. Another class of preferred esters are esters formed by the carboxyl group of the active ingredient of the present invention with an alcohol. An alcohol suitable for forming esters includes, but is not limited to: C1-C6 alkyl-OH, such as methanol, ethanol, n-propanol, isopropanol, etc.

The compound of the invention may be amorphous, crystalline or a mixture thereof.

In another preferred embodiment, each of R₁, R₂, R₃, R₄, R₅, R₆, R₇ and R₅ independently comprises the corresponding groups of compounds in Group A.

The compound of formula I of the invention can be extracted from plants by alcohol extraction, chromatography, etc., and can also be purchased through commercial channels or synthesized by using commercially available raw materials, or through the synthesis method in the prior art. Those skilled in the art can extract or synthesize the compound of the invention according to the prior well-known technology. The extract or synthetic compound can be further purified by column chromatography, high performance liquid chromatography or crystallization.

The methodology of synthetic chemical transformation and protection of functional groups is very helpful for the synthesis of applied compounds and is a well-known technology in the prior art. For example, see R. Larock, Comprehensive Organic Transformations, VCH Publishers(1989); T.W.Greene and P.G.M. Wuts, Protective Groups in Organic Synthesis, 3rd Ed., John Wiley and Sons(1999); L. Fieser and M.Fieser, Fieser and Fieser's Reagents for Organic Synthesis, John Wiley and Sons(1994); and L.Paquette, ed. Encyclopedia of Reagents for Organic Synthesis, John Wiley and Sons(1995), etc.

### Medicinal materials, food materials and extracts containing compounds of formula I

In another aspect of the present invention, it provides a use of a medicinal material and/or food material containing the compound of formula I, or an extract containing the compound of formula I for treating arterial diseases or for manufacturing a composition and/or formulation used for treatment of arterial diseases. Typically, the arterial diseases include, but are not limited to: (i) aneurysm; (ii) arterial intramural hematoma; and/or (iii) arterial dissection.

Some preferred compounds of the present invention are present in certain medicinal materials (e.g., Chinese herbal medicines) and food materials. Representative examples include, but are not limited to, ferulic acid, caffeic acid, Danshensu, phenethyl caffeinate, methyl rosmarinate, coumarin, p-coumarin acid, scopoletin, eugenol, and a combination thereof.

Some medicinal materials and food materials, including those from plants or animals or other sources, may contain one or more of the compound of the present invention.

Preferably, one or more compounds of the invention are the main active ingredients in the extracts from medicinal materials and food materials (for example, the content thereof is ≥ 0.1 wt% of the weight of the extract and preferably ≥ 0.5wt%).

One preferred medicinal material is propolis *(Colla Apis),* whose active ingredients contain phenethyl caffeate, in additional to ferulic acid, caffeic acid, *p*-coumaric acid and the like.

One preferred medicinal material or food material is peppermint *(Mentha haplocalyx Briq.),* whose active ingredients contain methyl rosmarinate, caffeic acid, ethyl rosmarinate, methyl caffeate, Danshensu and the like.

Other representative ferulic acid-containing medicinal materials include, but are not limited to, Ferulae Resina, Angelicae Sinensis Radix, Selaginellae Herba, Equiseti Hiemalis Herba, Allium Cepa, Chuanxiong Rhizoma, Cimicifugae Rhizoma, Propolis, Ziziphi Spinosae Semen, Flos Anisopappi Chinensis and a combination thereof.

Preferred Danshensu-containing medical materials include, but are not limited to Salviae Miltiorrhizae Radix Et Rhizoma, peppermint, Perillae Folium and a combination thereof.

Preferred caffeic acid-containing medical materials include, but are not limited to Cimicifugae Rhizoma, Daucus Carota L., Stachys Palustris L., Fagopyri Dibotryis Rhizoma, Elaeagnus Multiflora Thunb and a combination thereof.

Preferred phenethyl caffeate-containing medical materials include, but are not limited to various propolis.

Preferred methyl rosmarinate-containing medical materials include, but are not limited to Rabdosiae Rubescentis Herba, Vitex Negundo L., South Salviae Miltiorrhizae Radix Et Rhizoma, Rabdosia Serra, Perillae Folium and a combination thereof.

Preferred coumarin-containing medical materials include, but are not limited to *Fraxini Cortex, Artemisiae Scopariae Herba, Cnidii Fructus, Angelicae Pubescentis Radix, Daphne odora Thunb.,* and a combination thereof.

Preferred *p*-coumarin acid-containing medical materials include, but are not limited to *Hedyotis diffusa, propolis, Carthami Flos, Ginseng Radix Et Rhizoma, Cuscutae Semen, leaf of Juglans regia L.,* and a combination thereof.

Preferred eugenol-containing medical materials include, but are not limited to *Caryophylli Flos, Murrayae Folium Et Cacumen, Cinnamomum Purpureum, Alpinia Galanga (L.) Willd, Magnoliae Flos, Narcissus Tazetta L., Commiphora Myrrha Engl., Rosae Rugosae Flos, Thymus Mongolicus Ronn, Cestrum Nocturnum L.,* and a combination thereof.

Preferred ferulic acid-containing medical materials include, but are not limited to Ferulae Resina, coffea, chaff, vanilla bean, wheat bran, rice bran, peppermint, Perillae Folium and a combination thereof.

Preferred Danshensu-containing food materials include, but are not limited to Salviae Miltiorrhizae Radix Et Rhizoma, peppermint, Perillae Folium and a combination thereof.

Preferred caffeic acid-containing medical materials include, but are not limited to, coffea, buckwheat, *Artemisia ounghusbandii* and a combination thereof.

Preferred methyl rosmarinate-containing food materials include, but are not limited to *Menthae Haplocalycis Herba, Perillae Folium* and a combination thereof.

In the present invention, the medicinal material, food material or extract contains one or more compounds with the structure of formula I, therefore it can also be used for preventing and/or treating: (I) aneurysm; (II) arterial intramural hematoma; and/or (III) arterial dissection, and can be used to prepare pharmaceutical compositions or healthcare compositions.

### Pharmaceutical Composition And Administration Mode

The invention also provides a pharmaceutical composition useful for treating arterial diseases. The pharmaceutical composition contains a compound of formula I of the invention, or an isomer, a crystal form, a hydrate or a solvate thereof, or a pharmaceutically acceptable salt thereof as an active ingredient. The pharmaceutical composition of the invention has excellent effects of preventing and/or treating aneurysm, intramural hematomas and/or arterial dissections.

In the invention, the compound of formula I can be used in the form of pure substance, extract (or mixture), or directly in the form of medicinal materials (or food materials).

The pharmaceutical composition of the invention can be a single prescription (containing one active ingredient) or a compound prescription (containing two or more active ingredients).

In another preferred embodiment, the pharmaceutical composition of the invention comprises: (a) a first active ingredient selected from the group consisting of: a compound of formula I, an isomer thereof, a crystal form thereof, a hydrate or solvate thereof, or a pharmaceutically acceptable salt thereof, and a combination thereof; (b) a second active ingredient selected from the group consisting of: polymeric salvianolic acid, a stereoisomer, a crystal form, a pharmaceutically acceptable salt thereof, and a combination thereof; and (c) a pharmaceutically acceptable carrier.

Preferably, said first active ingredient (or optionally second active ingredient) can be added into the pharmaceutical composition in the form of an extract comprising the same.

According to the invention, the first active ingredient and the second active ingredient may be manufactured into two separate preparations, respectively, or mixed together to make a preparation. The two separate preparations can be administered simultaneously, separately or sequentially.

The "safe and effective amount" refers to that the amount of the compound is sufficient to significantly improve the condition without causing serious side effects. Typically, the pharmaceutical composition contains 1-2000 mg of the compound of the present invention per dose, and more preferably, contains 10-500 mg of the compound of the present invention per dose. Preferably, the "per dose" or one dose is a capsule or a tablet.

The "pharmaceutically acceptable carrier" refers to one or more compatible solid or liquid fillers or gel materials, which are suitable for human use, and must be of sufficient purity and sufficiently low toxicity. The "compatible" herein refers to that each component in the composition can be mixed with the compound of formula I without significantly reducing the efficacy of the compound. Some examples of pharmaceutically acceptable carrier include cellulose and its derivatives (such as sodium carboxymethyl cellulose, sodium ethyl cellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween ^{®}), wetting agent (such as sodium dodecyl sulfate), colorant, flavor, stabilizer, antioxidant, preservative, pyrogen free water and the like.

There are no special restrictions on the administration methods of the pharmaceutical composition of the invention, and the representative administration methods include but are not limited to: oral, rectal, parenteral (intravenous, intramuscle or subcutaneous), and topical administration.

Solid dosage forms for oral administration include capsules, tablets, pills, powders, and granules. Among these solid dosage forms, the active compound is mixed with at least one conventional inert excipient or carrier such as sodium citrate or calcium phosphate, or one or more following components: (a) filler or compatibilizer, for example, starch, lactose, sucrose, glucose, mannitol, and silicic acid; (b) binder, for example, hydroxymethyl cellulose, alginate, gelatin, polyvinylpyrrolidone, sucrose and arabic gum; (c) humectant, for example, glycerin; (d) disintegrant, for example, agar, calcium carbonate, potato starch or cassava starch, alginate, certain composite silicate, and sodium carbonate; (e) retarding solvent, such as paraffin; (f) absorption accelerator, for example, quaternary amine compound; (g) wetting agent, such as cetyl alcohol and glyceryl monostearate; (h) adsorbent, for example, kaolin; and (i) lubricants, for example, talc, calcium stearate, magnesium stearate, solid polyethylene glycol, sodium dodecyl sulfate, or mixtures thereof. In capsules, tablets and pills, the formulation may also include a buffer.

Solid dosage forms, e.g. tablets, sugar pills, capsules, pills and granules, may be prepared using coating and shell materials, such as casings and other materials known in the art. They may comprise an opacifying agent, and the release of the active ingredient in such a composition may be released in a delayed manner in a part of the digestive tract. Examples of embedding components that can be employed are polymeric substances and wax substances. If necessary, the active compound may also form a microcapsule form with one or more of the excipients described above.

Liquid dosage forms for oral administration include pharmaceutically acceptable emulsions, solutions, suspensions, syrups or tinctures. In addition to the active ingredients, the liquid dosage forms may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil, or a mixture thereof.

In addition to these inert diluents, the composition may also contain auxiliaries such as wetting agents, emulsifiers, suspending agents, sweeteners, flavoring agents and flavors.

In addition to the active ingredient, the suspension may comprise suspending agents, such as ethoxylated isooctadecanol, polyoxyethylene sorbitol and dehydrated sorbitol esters, microcrystalline cellulose, methanolic aluminum, agar, and any mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

The dosage forms for topical administration of the compound of the present invention include ointments, powder, patches, propellants and inhalants. The active ingredient is mixed under sterile conditions with a physiologically acceptable carrier and any preservatives buffers or propellants as may be required.

The active ingredient of the invention may be administered alone, or in combination with other pharmaceutically acceptable compounds, which includes, but not limited to, hypotensive agents (e.g. angiotensin-converting enzyme inhibitors and/or angiotensin receptor blockers), hypolipidemic agents (e.g. statins, fibrates, nicotinic acid and cholesterol absorption inhibitors), hypoglycemic agents (e.g. sulfonylurea secretagogues, insulin sensitizers, biguanides), polymeric salvianolic acids, and a combination thereof.

In the present invention, the general range of the therapeutic effective dose of the compound of formula I will be: about 1-2000mg/day, about 10-about 1000mg/day, about 10-about 500mg/day, about 10-about 250mg/day or about 10-about 100mg/day. The therapeutically effective dose will be given in one or more dosages. However, it should be understood that a particular dose of the active ingredient of the invention for any particular patient will depend on a number of factors, such as, for example, age, gender, weight, general health, diet, individual response of the patient to be treated, time of administration, severity of the disease to be treated, activity of the administered specific compound, dosage form, mode of application, and concomitant medication. The therapeutic effective dose of a given situation can be determined by routine experiments and is within the clinician or physician's ability and judgment. In any case, the active ingredient will be administered in multiple doses based on the individual condition of the patient and in a manner that allows delivery of a therapeutically effective amount.

### The main advantages of the present invention include:

1. It has been found for the first time in the present invention that the compound of formula I has the effect of slowing the expansion of aneurysm, reducing the arterial intramural hematoma, inhibiting arterial dissection, thus reducing the arterial rupture, and is suitable for treating artery-related diseases.
2. The compound of the invention can not only alleviate the expansion of early and mid-term aneurysms, but also has obvious effect on treating late aneurysms, can reduce the rupture risk of late aneurysms, and can be used for aneurysms in all phases.
3. The compound and pharmaceutical composition of the invention provides therapeutic options, in addition to surgery, for patients with aneurysms, intramural hematomas, and/or arterial dissections.
4. The compound of the present invention has good safety and little toxic or side effects.
5. The compounds of the present invention are simple in structure, can be easily synthesized or extracted from Chinese herbal medicines.
6. The compounds of the present invention are abundant in certain medicinal materials or food materials, including certain safe and easily available Chinese medicinal materials that are also used as food material,. The present invention, for the first time, validate the effects of propolis and peppermint (or extracts thereof) in the treatment of arterial diseases, including aneurysms.

The present invention will be further explained below in conjunction with specific embodiments. It should be understood that these embodiments are only used to illustrate the present invention and not to limit the scope of the present invention. In the following examples, the test methods without specific conditions are usually in accordance with conventional conditions or the conditions recommended by the manufacturer. Unless otherwise specified, percentages and parts are percentages by weight and parts by weight.

**Reagents**

| reagent | Source | Purity |
|---|---|---|
| Porcine pancreatic elastase | Sigma | 6.8U/ml |
| Phosphate buffer | Sinopharm Chemical ReagentCo., Ltd. | |
| Propolis | Jiangsu Beevip Biotechnology Co., Ltd. | |
| Peppermint powder | Nanjing Xinhou Biotechnology Co., Ltd. | |
| Phenethyl caffeate | Shanghai U-see Biotech Co., Ltd. | 99% |
| Ferulic acid | Shanghai U-see Biotech Co., Ltd | 99% |
| Piperazine ferulate | HUNAN QIAN JIN XIANGJIANG PHARMAGEUICAL CO. LTD. | Tablet |
| Ferulic acid sodium | Dalian Meilun Biotech Co., Ltd. | 99% |
| Methyl rosmarinate | Shanghai U-see Biotech Co., Ltd | 99% |
| Caffeic acid | Shanghai U-see Biotech Co., Ltd | 99% |
| Eugenol | Shanghai Saiyi Biotechnology Co., Ltd. | 99% |
| Danshensu | Shanghai U-see Biotech Co., Ltd | 99% |
| Coumarin | Wuhan Yuancheng Gongchuang Technology Co., Ltd. | 99% |
| *p*-Coumaric acid | Wuhan Yuancheng Gongchuang Technology Co., Ltd. | 99% |
| Scopoletin | Chengdu Herbpurify Co., Ltd. | 99% |
| Carvacrol | Nanjing Xinhou Biotechnology Co., Ltd. | 99% |
| Paeonol | Dalian Meilun Biotech Co., Ltd. | 99% |
| Aspirin eugenol ester | Shanghai U-see Biotech Co., Ltd. | 99% |
| Sivelestat sodium | Dalian Meilun Biotech Co., Ltd. | 99% |

**Experimental Instruments**

| Instrument | Manufacturer | Model |
|---|---|---|
| Automatic dehydrator | Leica, Germany | TP1020-3 |
| Embedding machine | Leica, Germany | EG1150H |
| Paraffin microtome | Leica, Germany | RM2016 |
| Slide spreader | Leica, Germany | HI1210 |
| Slide drier | Leica, Germany | HI1220 |
| Cold plate | Leica, Germany | EG1150C |
| Stereoscopic microscope | OLYMPUS, Japan | BX-51 |
| Fluorescent microscopy | OLYMPUS, Japan | SZX7 |
| Clean bench | Suzhou Sewage Treatment Co., Ltd. | SW-CJ-1F |
| Centrifuge | Xiang Yi Laboratory Instrument Development Co., Ltd. | L-500 |
| Vortex shaker | Shanghai Qite Analytical Instrument Co., Ltd. | QT-1 |
| Electronic scale | Sartorius, Germany | CP124S |
| Electronic scale | Sartorius, Germany | BT125D |
| Pressure steam sterilizer | Shanghai Shen'an Medical Appliance Factory | LDZX-75KBS |

### Model preparation and sample detection methods

### Aneurysm model preparation

The mouse was anesthetized by intraperitoneal injection of 1% pentobarbital sodium at the dose of 20mg/kg. After the mouse entering anesthesia state, the abdominal hair was shaved off with a razor and further thoroughly depilated with depilatory cream. The abdomen was dried and the mouse was fixed on the operating table. Its body temperature was kept with a heating pad under the body. The abdominal skin was wiped with iodine and deiodined with 75% ethanol. An incision of about 1.5 cm was cut in the middle of the abdomen. The organs were carefully pushed away with tweezers. Then the intestine was divided to the left and right sides with gauze wetted with 3% penicillin-streptomycin. The connective tissue and muscle tissue on the surface of the aorta were carefully separated with tweezers to fully expose the abdominal aorta. The length of exposed abdominal aorta was about 0.5 cm, whose proximal end did not exceed the bilateral renal arteries and distal end did not exceed femoral arteries.

Sterilized absorbent paper (0.3cm × 0.3cm) was fully infiltrated in porcine pancreatic elastase (PPE), and then applied on the exposed abdominal aorta for 50 minutes. A gauze wetted with normal saline containing 3% penicillin-streptomycin was placed on the abdominal incision to prevent the abdominal water from excessive loss. After 50 minutes, the absorbent paper on the aorta was carefully taken away. The abdominal cavity was rinsed with normal saline containing 3% penicillin-streptomycin. The peritoneum and skin were sutured with 3/8 suture needle and 5/0 suture line. After suturing, the wound was wiped with iodine and deiodined with 75% ethanol after 1 min. The mouse was placed into a clean feeding cage and can eat and drink freely.

### Aneurysm, intermural hematoma and arterial dissection model preparation

The mouse was anesthetized by intraperitoneal injection of 1% pentobarbital sodium at the dose of 20mg/kg. After the mouse entering anesthesia state, the abdominal hair was shaved off with a razor and further thoroughly depilated with depilatory cream. The abdomen was dried and the mouse was fixed on the operating table. Its body temperature was kept with a heating pad under the body. The abdominal skin was wiped with iodine and deiodined with 75% ethanol. An incision of about 1.5 cm was cut in the middle of the abdomen. The organs were carefully pushed away with tweezers. Then the intestine was divided to the left and right sides with gauze wetted with 3% penicillin-streptomycin. The connective tissue and muscle tissue on the surface of the aorta were carefully separated with tweezers to fully expose the abdominal aorta. The length of exposed abdominal aorta was about 0.5 cm, whose proximal end did not exceed the bilateral renal arteries and distal end did not exceed femoral arteries.

Sterilized absorbent paper (0.3cm × 0.3cm) was fully infiltrated in porcine pancreatic elastase (PPE), and then applied on the exposed abdominal aorta for 50 minutes. A gauze wetted with normal saline containing 3% penicillin-streptomycin was placed on the abdominal incision to prevent the abdominal water from excessive loss. After 50 minutes, the absorbent paper on the aorta was carefully taken away. And the abdominal cavity was rinsed with normal saline containing 3% penicillin-streptomycin. The peritoneum and skin were sutured with 3/8 suture needle and 5/0 suture line. After suturing, the wound was wiped with iodine and deiodined with 75% ethanol after 1 min. The mouse was placed into a clean feeding cage, fed with 1% β-aminopropionitrile (BAPN) feed and can eat and drink freely. The feed was changed every other day.

### Sample fixation, dehydration, paraffin embedding and slicing

100 ml of formaldehyde, 4 g of sodium dihydrogen phosphate and 6.5 g of disodium hydrogen phosphate were dissolved in 900 ml of distilled water to prepare a polyoxymethylene fixed solution with a volume ratio of 10%. The aortic tissue was fixed in polyoxymethylene fixation solution for 72 hours, then washed with tap water for 4-6 hrs and placed in the dehydrator. The program was set for automatic dehydration, with 75% ethanol for 1.5 hours, 95% ethanol for 1.5 hours, 100% ethanol for 1.5 hours, dimethylbenzene for 1.5 hours and paraffin for 1.5 hours, successively. The paraffin embedding machine was opened 2 hours in advance to melt paraffin, and the temperature was controlled at 60 °C. After the paraffin was melted, the dehydrated aortic tissue was embedded in paraffin. It was poured into the embedding box, and the paraffin soaked tissue block was placed into the embedding frame using heated tweezers and gently moved to the cold table. The paraffin block was taken off for slicing after the paraffin was solidified. Before slicing, the paraffin block was put into refrigerator for pre-cooling. After cooling, the paraffin block was cut into continuous 5 µM-thick paraffin sections using the slicer. The sections were unfolded on warm water at 38 °C in a slide spreader, fished with slides coated with APES, and dried naturally for subsequent histopathological staining.

### Hematoxylin-Eosin Staining

The tissue was fixed, embedded and cut into 4 µM paraffin sections. The paraffin sections were firstly baked (65 °C, 60 minutes), and then dewaxed to aqueous phase (dimethylbenzene for 15 minutes → absolute ethanol for 5 minutes → 95% ethanol for 5 minutes → 75% ethanol for 5 minutes → running water for 1 minute); stained with hematoxylin for 15 minutes and then rinsed with running water for 4 minutes; differentiated with 1% ethanol hydrochloride for 5 seconds (the differentiation time was adjusted according to the preparation time of differentiation solution) and rinsed with running water for 5 minutes; then stained with eosin for 1 minute and placed in water for 1 minute; dehydrated and then permeated with dimethylbenzene (75% ethanol for 5 minutes → 95% ethanol for 5 minutes → absolute ethanol for 5 minutes → dimethylbenzene for 15 minutes) and sealed with neutral gum. BX51 microscope was used to taken photos.

### Masson Staining

Paraffin sections were baked at 65 °C for 60 minutes and then dewaxed to aqueous phase (dimethylbenzene for 15 minutes → absolute ethanol for 5 minutes → 95% ethanol for 5 minutes → 75% ethanol for 5 minutes → running water for 1 minute); treated with chrome or mercury-removal salt precipitation, and then washed by tap water and distilled water in turn. The nuclei were stained with Harris hematoxylin or Weigert hematoxylin for 1-2 minutes. It was fully washed with water. If it was over stained, it could be differentiated with hydrochloric acid and alcohol for 2-3 seconds. Warm water was used to return the color to blue. Masson ponceau acid reddish staining solution was used to stain for 5-10 minutes. The sample was then differentiated with 1% phosphomolybdic acid aqueous solution for 3-5min (microscopicly observing the coloring situation), stained with 1% aniline blue or light green solution for 5 minutes, differentiated with 1% glacial acetic acid aqueous solution for several seconds (microscopicly observing the coloring situation) and then permeated with dimethylbenzene (75% ethanol for 5 minutes → 95% ethanol for 5 minutes → absolute ethanol for 5 minutes → dimethylbenzene for 15 minutes) and sealed with neutral gum. BX51 microscope was used to taken photos.

### Orcein Staining

The tissue was fixed, embedded and cut into 4µm paraffin sections. The paraffin sections were firstly baked (65 °C, 60 minutes), and then dewaxed to aqueous phase (dimethylbenzene for 15 minutes → absolute ethanol for 5 minutes → 95% ethanol for 5 minutes → 75% ethanol for 5 minutes → running water 1 minute); stained with orcein for 60 minutes and differentiated with 1% ethanol hydrochloride for 5 minutes, then rinsed with distilled water and sealed with neutral gum. BX51 microscope was used to taken photos for Statistical analysis.

### Prussian Blue Staining

The tissue was fixed, embedded and cut into 4µm paraffin sections. The paraffin sections were firstly baked (65 °C, 60 minutes), and then dewaxed to aqueous phase (dimethylbenzene for 15 minutes → absolute ethanol for 5 minutes 95% ethanol for 5 minutes → 75% ethanol for 5 minutes → running water for 1 minute); stained with prussian blue for 60 minutes and with added nuclear fast red solution for 5 minutes, then rinsed with distilled water and sealed with neutral gum. BX51 microscope was used to taken photos.

### Immunohistochemical Staining

The slides were soaked in acetone solution containing 10% APES for 30 minutes, then dried in a fume hood. The aortic tissue was cut into 4µm wax pieces and was pasted on the slide smoothly. Paraffin sections were baked at 65 °C for 60 minutes and then dewaxed to aqueous phase (dimethylbenzene for 15 minutes → absolute ethanol for 5 minutes → 95% ethanol for 5 minutes → 75% ethanol for 5 minutes → running water for 1 minute). The tissue was placed in sodium citrate repair solution (pH = 6.0) for microwave repair for 20 minutes (96 °C), removed and cooled to room temperature, and rinsed with PBS for 3 times for 5 minutes each time, incubated with 3% hydrogen peroxide at room temperature for 15 minutes(in order to inactivate the endogenous peroxidase), then rinsed with PBS for 3 times for 5 minutes each time; added with 10% normal goat serum blocking solution and incubated at 37 °C for 30 minutes. Then the blocking solution was removed and the primary antibody (CD68) was added. The sample was then incubated in the refrigerator at 4 °C overnight; rinsed with PBS for 5 times for 5 minutes each time; then added secondary antibody (Goat anti-rabbit antibody) and incubated at 37 °C for 1 hour; rinsed with PBS for 5 times for 5 minutes each time, then added with fresh DAB working solution, developed for 2 minutes and rinsed with water for 3 minutes; restained with Hematoxylin for 15 minutes; differentiated with 1% ethanol hydrochloride for 5 seconds (the differentiation time was adjusted according to the preparation time of differentiation solution), and rinse with running water for 5 minutes, then permeated with dimethylbenzene (75% ethanol for 5 minutes → 95% ethanol for 5 minutes → absolute ethanol for 5 minutes → dimethylbenzene for 15 minutes) and sealed with neutral gum. BX51 microscope was used to taken photos.

### Statistical Analysis

Data were expressed as mean ± SE. Graphpad Prism 6 was used for statistical analysis. The statistical significance of the differences between the groups was compared for multiple times by one-way analysis of variance. After determining the normal distribution and homogeneity of variance, Tukey test was carried out. P < 0.05 represents statistically significant.

### Example 1

### The preventive and therapeutic effect of ferulic acid on aneurysm, intermural hematoma, and arterial dissection

### (1) The preventive and therapeutic effect of ferulic acid on aneurysm

**Administration and grouping of animals:** 30 8-week aged C57BL/6 mice were adaptive fed for 1 week in animal room, and then randomly divided into normal control group, model control group and ferulic acid group (n=10 for each group). Aneurysms were induced by porcine pancreatic elastase. The day of surgery was set as Day 0. Saline or ferulic acid were administered to animals by intraperitoneal injection from Day 5 and the dosage of ferulic acid was 20mg/kg. The drug was continuously administered for 10 days and the activities of the animals were closely observed. Animals were euthanized on Day 15 and the arterial tissues were obtained to evaluate the expansion of aneurysm.

### Experimental results and analysis:

The experimental results are shown in Figures 1A-C. It can be seen that compared with the mice in normal control group, the infrarenal arteries of the model control group obviously bulged and expanded. Ferulic acid can significantly reduce the diameter of aneurysms in mouse (P<0.001), showing a significant effect of preventing and treating aneurysm.

### (2) The preventive and therapeutic effect of ferulic acid on aneurysm, intermural hematoma, and arterial dissection

**Administration and grouping of animals:** 30 8-week aged C57BL/6 mice were adaptive fed for 1 week in animal room, and then randomly divided into normal control group, model control group and ferulic acid group(n=10 for each group). Aneurysms, intramural hematomas and arterial dissections were induced by porcine pancreatic elastase and 1% β-aminopropionitrile. The day of surgery was set as Day 0. Saline or ferulic acid was administered to animals by intraperitoneal injection from Day 5 and the dosage of ferulic acid was 100mg/kg. The drug was continuously administered for 10 days and the activities of the animals were closely observed. Animals were euthanized on Day 15 and the arterial tissues were obtained to evaluate the artery intramural hematoma and arterial dissection rupture.

**Experimental results and analysis:** By using models of aneurysms, intramural hematomas and arterial dissections induced by feeding porcine pancreatic elastase and 1% β-aminopropionitrile, the effect of the drugs on aneurism development and intramural hematoma and arterial dissection rupture could be studied. The experimental results are shown in Figures 1D-G. It could be seen that ferulic acid significantly reduced the expansion of aneurysm in mouse (P < 0.001), and also significantly reduced the occurrence of intramural hematoma and arterial dissection, and the arterial rupture in mouse. Histological analysis showed that ferulic acid protected the integrity of vessel structure, inhibited the degradation of elastic layer, inhibited the occurrence of intramural hematoma, protected the morphology of vascular adventitia collagen and reduced the risk of vascular tear and rupture.

### Example 2

### The preventive and therapeutic effect of piperazine ferulate (via oral intragastric administration) on aneurysm, intermural hematoma, and arterial dissection

**Administration and grouping of animals:** 30 8-week aged C57BL/6 mice were adaptive fed for 1 week in animal room, and then randomly divided into normal control group, model control group and piperazine ferulate group (n=10 for each group). Intramural hematomas and arterial dissections were induced by porcine pancreatic elastase and 1% β-aminopropionitrile. The day of surgery was set as Day 0. 0.5% carboxymethyl cellulose sodium or piperazine ferulate was orally administered to animals via gavage from Day 5 and the dosage of piperazine ferulate was 50mg/kg. The drug was continuously administered for 10 days and the activities of the animals were closely observed. Animals were euthanized on Day 15 and the arterial tissues were obtained to evaluate the artery intramural hematoma and arterial dissection.

**Experimental results and analysis:** The experimental results are shown in Figure 2. It could be seen that compared with the mice in normal control group, piperazine ferulate significantly reduced the expansion of arteries in mouse(P < 0.001), and also significantly reduced the occurrence of aneurysm, intramural hematoma and arterial dissection, reduced the portion of arterial rupture and inhibited aortic dissection in mouse. Histological analysis showed that piperazine ferulate reduced the thickness of vessel wall and protected the integrity of vessel structure.

### Example 3

### (1) The preventive and therapeutic effect of Danshensu on preventing and treating aneurysm

**Administration and grouping of animals:** 30 8-week aged C57BL/6 mice were adaptive fed for 1 week in animal room, and then randomly divided into normal control group, model control group and Danshensu group (n=10 for each group). Aneurysms were induced by porcine pancreatic elastase. The day of surgery was set as Day 0. Saline or Danshensu was administered to animals by intraperitoneal injection from Day 5 and the dosage of Danshensu was 20mg/kg. The drug was continuously administered for 10 days and the activities of the animals were closely observed. Animals were euthanized on Day 15 and the arterial tissues were obtained to evaluate the expansion of aneurysm.

**Experimental results and analysis:** The experimental results are shown in Figure 3. It could be seen that compared with the mice in normal control group, the infrarenal arteries of the model control group obviously bulged and expanded. Danshensu significantly reduced the diameter and expansion of aneurysm (P<0.01), showing a significant effect of preventing and treating aneurysm.

### Example 4

### (1) The preventive and therapeutic effect of caffeic acid on aneurysm

**Administration and grouping of animals:** 30 8-week aged C57BL/6 mice were adaptive fed for 1 week in animal room, and then randomly divided into normal control group, model control group and caffeic acid group (n=10 for each group). Aneurysms were induced by porcine pancreatic elastase. The day of surgery was set as Day 0. Saline or caffeic acid was administered to animals by intraperitoneal injection from Day 5 and the dosage of caffeic acid was 20mg/kg. The drug was continuously administered for 10 days and the activities of the animals were closely observed. Animals were euthanized on Day 15 and the arterial tissues were obtained to evaluate the expansion of aneurysm.

**Experimental results and analysis:** The experimental results are shown in Figure 4. It could be seen that compared with the mice in normal control group, the infrarenal arteries of the model control group obviously bulged and expanded. Caffeic acid significantly reduced the diameter and expansion of aneurysm in mouse (P<0.01), showing a significant effect of preventing and treating aneurysm.

### Example 5

### The preventive and therapeutic effect of phenethyl caffeate on aneurysm, intermural hematoma, and arterial dissection

### (1) The preventive and therapeutic effect of phenethyl caffeate on aneurysm

**Administration and grouping of animals:** 30 8-week aged C57BL/6 mice were adaptive fed for 1 week in animal room, and then randomly divided into normal control group, model control group and phenethyl caffeate group (n=10 for each group). Aneurysms were induced by porcine pancreatic elastase. The day of surgery was set as Day 0. 0.5% carboxymethyl cellulose sodium or phenethyl caffeate was orally administered to animals via gavage from Day 5 and the dosage of phenethyl caffeate was 100mg/kg. The drug was continuously administered for 10 days and the activities of the animals were closely observed. Animals were euthanized on Day 15 and the arterial tissues were obtained to evaluate the expansion of aneurysm.

**Experimental results and analysis:** The experimental results are shown in Figure 5A-C. It could be seen that compared with the mice in normal control group, the infrarenal arteries of the model control group obviously bulged and expanded. Phenethyl caffeate significantly reduced the diameter of infrarenal aneurysms in mouse (P<0.001), showing a significant effect of preventing and treating aneurysm.

### (2) The preventive and therapeutic effect of phenethyl caffeate on aneurysm, intermural hematoma, and arterial dissection

**Administration and grouping of animals:** 30 8-week aged C57BL/6 mice were adaptive fed for 1 week in animal room, and then randomly divided into normal control group, model control group and phenethyl caffeate group(n=10 for each group). Intramural hematomas and arterial dissections were induced by porcine pancreatic elastase and 1% β-aminopropionitrile. The day of surgery was set as Day 0. Saline or phenethyl caffeate was administered to animals by intraperitoneal injection from Day 5 and the dosage of phenethyl caffeate was 100mg/kg. The drug was continuously administered for 10 days and the activities of the animals were closely observed. Animals were euthanized on Day 15 and the aorta tissues were obtained to evaluate the artery intramural hematoma and arterial dissection.

**Experimental results and analysis:** The experimental results are shown in Figures 5D-G. It could be seen that phenethyl caffeate significantly reduced the diameter of artery expansion in mouse (P < 0.01) and also significantly reduced the occurrence of intramural hematoma and arterial dissection, and the arterial rupture in mouse. Histological analysis showed that phenethyl caffeate reduced the thickness of vessel wall and protected the integrity of vessel structure, inhibited the degradation of elastic layer, inhibited the occurrence of intramural hematoma and protected the morphology of vascular adventitia collagen.

### Example 6

### The preventive and therapeutic effect of methyl rosmarinate on aneurysm, intermural hematoma, and arterial dissection

**Administration and grouping of animals:** 30 8-week aged C57BL/6 mice were adaptive fed for 1 week in animal room, and then randomly divided into normal control group, model control group and methyl rosmarinate group (n=10 for each group). Intramural hematomas and arterial dissections were induced by porcine pancreatic elastase and 1% β-aminopropionitrile. The day of surgery was set as Day 0. Saline or methyl rosmarinate was administered to animals by intraperitoneal injection from Day 5 and the dosage of methyl rosmarinate was 100mg/kg. The drug was continuously administered for 10 days and the activities of the animals were closely observed. Animals were euthanized on Day 15 and the arterial tissues were obtained for evaluation.

**Experimental results and analysis:** The experimental results are shown in Figure 6. It could be seen that methyl rosmarinate significantly reduced the occurrence of infrarenal aneurism, intramural hematoma and arterial dissection in mouse, thereby reducing arterial rupture. Histological analysis showed that methyl rosmarinate reduced the thickness of vessel wall and protected the integrity of vessel structure, inhibited the degradation of elastic layer, inhibited the occurrence of intramural hematoma and protected the morphology of vascular adventitia collagen.

### Example 7

### The preventive and therapeutic effect of eugenol (via intraperitoneal injection) on aneurysm, intermural hematoma, and arterial dissection

**Administration and grouping of animals:** 30 8-week aged C57BL/6 mice were adaptive fed for 1 week in animal room, and then randomly divided into normal control group, model control group and eugenol group (n=10 for each group). Intramural hematomas and arterial dissections were induced by porcine pancreatic elastase and 1% β-aminopropionitrile. The day of surgery was set as Day 0. Saline or eugenol was administered to animals by intraperitoneal injection from Day 5 and the dosage of eugenol was 100mg/kg. The drug was continuously administered for 10 days and the activities of the animals were closely observed. Animals were euthanized on Day 15 and the arterial tissues were obtained for evaluation.

**Experimental results and analysis:** The experimental results are shown in Figure 7. It could be seen that eugenol significantly reduced the diameter of infrarenal aneurysm in mouse(P < 0.01), thereby reducing the occurrence of infrarenal artery intramural hematoma and arterial dissection and the arterial rupture in mouse.

### Example 8

### The preventive and therapeutic effect of eugenol (via oral intragastric administration) on aneurysm, intermural hematoma, and arterial dissection

**Administration and grouping of animals:** 15 8-week aged C57BL/6 mice were adaptive fed for 1 week in animal room, and then randomly divided into normal control group, model control group and intragastric eugenol group (n=5 for each group). Intramural hematomas and arterial dissections were induced by porcine pancreatic elastase and 1% β-aminopropionitrile. The day of surgery was set as Day 0. 0.5% carboxymethyl cellulose sodium or eugenol was Intragastric administered to animals from Day 5 and the dosage of eugenol was 100mg/kg. The drug was continuously administered for 10 days and the activities of the animals were closely observed. Animals were euthanized on Day 15 and the expansion of aneurysm was evaluated.

**Experimental results and analysis:** The experimental results are shown in Figure 8. It could be seen that intragastric administered eugenol significantly reduced the diameter of infrarenal aneurysm in mouse(P <0.001), thereby reducing the occurrence of infrarenal artery intramural hematoma and arterial dissection in mouse.

### Example 9

### The preventive and therapeutic effect of aspirin eugenol ester on aneurysm, intermural hematoma, and arterial dissection

**Administration and grouping of animals:** 8-week aged C57BL/6 mice were adaptive fed for 1 week in animal room, and then randomly divided into normal control group, model control group and aspirin eugenol ester group. Aneurysms, intramural hematomas and arterial dissections were induced by feeding porcine pancreatic elastase and 1% β-aminopropionitrile. The day of surgery was set as Day 0. Saline or aspirin eugenol ester was administered to animals by intraperitoneal injection from Day 5 with the dosage of 100mg/kg. The drug was continuously administered for 10 days and the activities of the animals were closely observed. Animals were euthanized on Day 15 and the arterial tissues were obtained for evaluation.

**Experimental results and analysis:** The experimental results are shown in Figure 9. It could be seen that aspirin eugenol ester significantly reduced the occurrence of infrarenal artery intramural hematoma and arterial dissection in mouse(P <0.001). Histological analysis showed that aspirin eugenol ester reduced the thickness of vessel wall and protected the integrity of vessel structure.

### Example 10

### The preventive and therapeutic effect of carvacrol on aneurysm, intermural hematoma, and arterial dissection

**Administration and grouping of animals:** 8-week aged C57BL/6 mice were adaptive fed for 1 week in animal room, and then randomly divided into normal control group, model control group and carvacrol group. Intramural hematomas and arterial dissections were induced by feeding porcine pancreatic elastase and 1% β-aminopropionitrile. The day of surgery was set as Day 0. Saline or carvacrol was administered to animals by intraperitoneal injection from Day 5 with the dosage of 100mg/kg. The drug was continuously administered for 10 days and the activities of the animals were closely observed. Animals were euthanized on Day 15 and the arterial tissues were obtained for evaluation.

**Experimental results and analysis:** The experimental results are shown in Figure 10. It could be seen that carvacrol significantly reduced the diameter of infrarenal aneurysm in mouse (P < 0.001) and reduced the occurrence of infrarenal aneurysm, intramural hematoma and arterial dissection in mouse. Histological analysis showed that carvacrol reduced the thickness of vessel wall and protected the integrity of vessel structure.

### Example 11

### The preventive and therapeutic effect of paeonol on aneurysm, intermural hematoma, and arterial dissection

**Administration and grouping of animals:** 8-week aged C57BL/6 mice were adaptive fed for 1 week in animal room, and then randomly divided into normal control group, model control group and paeonol group. Aneurysms, intramural hematomas and arterial dissections were induced by feeding porcine pancreatic elastase and 1% β-aminopropionitrile. The day of surgery was set as Day 0. Saline or paeonol were administered to animals by intraperitoneal injection from Day 5 with the dosage of 100mg/kg. The drug was continuously administered for 10 days and the activities of the animals were closely observed. Animals were euthanized on Day 15 and the arterial tissues were obtained for evaluation.

**Experimental results and analysis:** The experimental results are shown in Figure 11. It could be seen that paeonol significantly reduced the diameter of infrarenal aneurysm in mouse (P < 0.001) and reduced the occurrence of infrarenal artery intramural hematoma and arterial dissection in mouse. Histological analysis showed that paeonol reduced the thickness of vessel wall and protected the integrity of vessel structure.

### Example 12

### The preventive and therapeutic effect of coumarin and p-coumaric acid on aneurysm, intermural hematoma, and arterial dissection

**Administration and grouping of animals:** 8-week aged C57BL/6 mice were adaptive fed for 1 week in animal room, and then randomly divided into normal control group, model control group, coumarin and *p*-coumaric acid group. Aneurysms, intramural hematomas and arterial dissections were induced by feeding porcine pancreatic elastase and 1% β-aminopropionitrile. The day of surgery was set as Day 0. 0.5% carboxymethyl cellulose sodium or coumarin or p-coumaric acid were intragastrically administered to animals from Day 5 with the dosage of 100mg/kg respectively. The drugs were continuously administered for 10 days and the activities of the animals were closely observed. Animals were euthanized on Day 15 and the arterial tissues were obtained to evaluate the expansion of aneurysm.

**Experimental results and analysis:** The experimental results are shown in Figure 12. It could be seen that both coumarin and *p*-coumaric acid significantly reduced the diameter of infrarenal aneurysm in mouse and inhibited the expansion of aortic aneurysms in mouse.

### Example 13

### The preventive and therapeutic effect of scopoletin on aneurysm, intermural hematoma, and arterial dissection

**Administration and grouping of animals:** 8-week aged C57BL/6 mice were adaptive fed for 1 week in animal room, and then randomly divided into normal control group, model control group and scopoletin group. Aneurysms, intramural hematomas and arterial dissections were induced by feeding porcine pancreatic elastase and 1% β-aminopropionitrile. The day of surgery was set as Day 0. 0.5% carboxymethyl cellulose sodium or scopoletin were intragastrically administered to animals from Day 5 with the dosage of 100mg/kg respectively. The drug was continuously administered for 10 days and the activities of the animals were closely observed. Animals were euthanized on Day 15 and the arterial tissues were obtained to evaluate the expansion of aneurysm.

**Experimental results and analysis:** The experimental results are shown in Figure 13. It could be seen that scopoletin significantly reduced the diameter of infrarenal aneurysm in mouse and inhibited the expansion of aortic aneurysms in mouse.

**Table 1 shows the structure and effect of each compound,**
wherein, for the aneurysm model induced by porcine pancreatic elastase (PPE model), "Ineffective" indicates no protective effect, "^{∗}" indicates a protective effect, "^{∗∗}" has a significant protective effect, and "^{∗∗∗}" indicates a very significant protective effect;
wherein, for the aneurysm model induced by porcine pancreatic elastase and 1% β-aminopropionitrile (PPE + BAPN model), "ineffective" indicates no protective effect, "^{∗}" indicates a protective effect, "^{∗∗}" has a significant protective effect, and "^{∗∗∗}" indicates a very significant protective effect.

**Table. 1**

| Drug name | Structure Formula | PPE | PPE+BAPN |
|---|---|---|---|
| Ferulic acid | 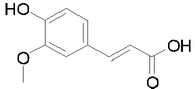 | *** | *** |
| Piperazine ferulate | 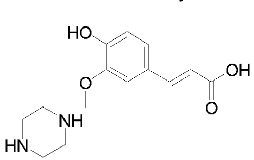 | / | *** |
| Caffeic acid | 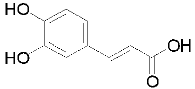 | ** | / |
| Phenethyl caffeate | 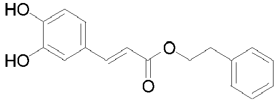 | *** | ** |
| Methyl rosmarinate | 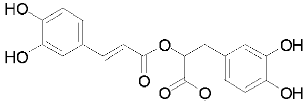 | *** | *** |
| Danshensu | 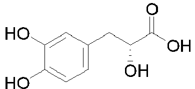 | ** | / |
| Coumarin | 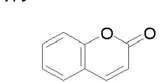 | / | * |
| *p*-Coumaric acid | 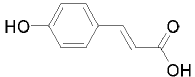 | / | * |
| Scopoletin | 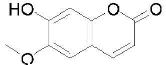 | / | * |
| Eugenol | 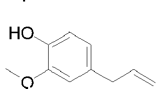 | / | *** |
| Carvacrol | 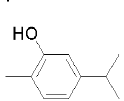 | / | *** |
| Paeonol | 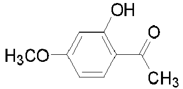 | / | *** |
| Aspirin eugenol ester | 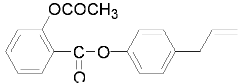 | / | *** |
| Sivelestat sodium | 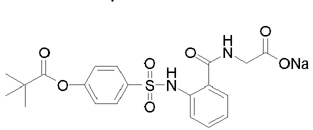 | ineffective | ineffective |

### Example 14

### The preventive and therapeutic effect of peppermint (Mentha haplocalyx Briq.) on aneurysm

In this example, peppermint powder, a material containing the compound of formula I, was studied for its effect of preventing and treating aneurysm. The main chemical components contained in peppermint are volatile oil, flavonoids, phenolic acids, anthraquinones and amino acids. Among the phenolic acids, methyl rosmarinate, caffeic acid, ferulic acid, ethyl rosmarinate and methyl caffeate are f compounds of formula I of the present invention.

**Administration and grouping of animals:** 30 8-week aged C57BL/6 mice were adaptive fed for 1 week in animal room, and then randomly divided into normal control group, model control group and peppermint powder (powder of the whole plant) group (n=10 for each group). Aneurysms were induced by porcine pancreatic elastase. The day of surgery was set as Day 0. 0.5% carboxymethyl cellulose sodium or peppermint powder was orally administered to animals via gavage from Day 5 and the dosage of peppermint powder was 200 mg/kg. The administration was continued for 10 days and the activities of the animals were closely observed. Animals were euthanized on Day 15 and the arterial tissues were obtained to evaluate the expansion of aneurysm.

**Experimental results and analysis:** The experimental results are shown in Figure 14. It could be seen that compared with the mice in normal control group, the infrarenal arteries of the model control group obviously bulged and expanded. Peppermint powder could significantly reduce the diameter of infrarenal aneurysms in mouse(P<0.01), showing a significant effect of preventing and treating aneurysm.

### Example 15

### Preventive and therapeutic effects of propolis (Colla Apis) on aneurysm

In this example, propolis, a material containing the compound of formula I, was studied for its effect of preventing and treating aneurysm. Propolis contains a lot of flavonoids, terpenes, esters, phenolic acids, sugars, a lot of amino acids and vitamins. The esters such as penethyl caffeate, benzyl caffeate, *p*-benzyl coumarin, *p*-phenethyl coumarin and cinnamyl caffeate , and phenolic acids such as ferulic acid, p-coumaric acid, long-chain caffeates, caffeic acid, o-coumaric acid are compounds of fomula I of the present invention.

**Administration and grouping of animals:** 30 8-week aged C57BL/6 mice were adaptive fed for 1 week in animal room, and then randomly divided into normal control group, model control group and propolis group (n=10 for each group). Aneurysms were induced by porcine pancreatic elastase. The day of surgery was set as Day 0. 0.5% carboxymethyl cellulose sodium or propolis was orally administered to animals via gavage from Day 5 and the dosage of propolis was 100 mg/kg. The administration was continued for 10 days and the activities of the animals were closely observed. Animals were euthanized on Day 15 and the arterial tissues were obtained to evaluate the expansion of aneurysm.

**Experimental results and analysis:** The experimental results are shown in Figure 15. It could be seen that compared with the mice in normal control group, the infrarenal arteries of the model control group obviously bulged and expanded. Propolis could significantly reduce the diameter of infrarenal aneurysms in mouse (P<0.001). Compared with mice in the normal control group, the vessel wall of the infrarenal artery of the model control group was significantly thickened, and a large number of macrophages infiltrated in the infrarenal abdominal aortic part, whereas the thickness of the infrarenal artery and macrophage infiltration of the mice were significantly reduced after the administration of propolis. As for the same dosage, the amounts of active components, such as phenethyl caffeate, ferulic acid, and caffeic acid, in propolis were much lower than those of pure compounds. Therefore, there are synergistic therapeutic effects of these compounds in propolis and/or synergistic therapeutic effects of these compounds together with other substances in propolis.

### Discussion

Aneurysm is due to lesions or damage of the arterial wall, causes localized or diffusely expanding or bulging of the arterial wall. Intramural hematoma is mainly characterized by hemorrhage in the middle layer of an artery. As the amount of blood increases, it can cause vessel rupture. Arterial dissection is the tearing of the arterial wall, and blood in vessels passes through the breach into the arterial wall to form a false lumen and the arterial wall then ruptures. Aneurysms, intramural hematomas, and arterial dissections are the main arterial related diseases that can lead to arterial rupture. They are closely related in the course of the disease and can affect or transform mutually. For example, aneurysms and intramural hematomas may evolve into arterial dissections, and intramural hematomas may lead to arterial dissections and ruptures. In the early stages of the diseases, aneurysms, arterial dissections, and intramural hematomas all exhibit, expansion of the arterial diameter with different degree,, and, in the advanced or late stages, all exhibit rupture of the artery, which endangers the patient's life.

Aneurysms can occur anywhere in the arterial system and include, but are not limited to, thoracic aneurysms, abdominal aneurysms, splenic aneurysms, hepatic aneurysms, superior mesenteric aneurysms, coeliac axis aneurysms, renal aneurysms, epiploon aneurysm, inferior mesenteric aneurysms, intracranial aneurysms, and carotid aneurysms.

Current treatments for aneurysms, arterial dissections and intramural hematomas are mainly surgery to prevent death due to the rupture. Globally, no new drugs have been developed for the prevention and treatment of aneurysms, arterial dissections, and intramural hematomas.

In this invention, persistent expansion of the arterial wall was induced by incubated with porcine pancreatic elastase to mimic the early and mid stage of aneurysms. Aneurysms, intramural hematomas, and vascular tearing was induced by incubating with porcine pancreatic elastase and feeding with β- aminopropionitrile feed to mimic the advanced stage of arterial dissections, intramural hematomas, and aneurysms.

In the present invention, it is unexpectedly observed that the compounds of the invention have therapeutic effects for early, middle and late aneurysm and are useful for the whole course of aneurysm. Arterial diseases mainly occur in the elderly. The advanced stages of arterial dissection, intramural hematoma and aneurysm are very likely to lead to the rupture of artery and endanger the patient's life. However, the surgical treatment and postoperative rehabilitation are also of great risk (especially for the elderly). Thus, the compounds of the invention also provide a new therapeutic method for patients with late aneurysm, which is of great significance.

All documents referred to in the present invention are incorporated by reference herein as if each document is individually incorporated by reference. Further, it should be understood that upon reading the above teaching of the present invention, various modifications or modifications may be made to the present invention by those skilled in the art, and those equivalents also fall within the scope defined by the appended claims of the present application.

## Claims

1. A use of a compound of formula I, or an isomer thereof, a crystal form thereof, a hydrate or a solvate thereof, or a pharmaceutically acceptable salt or ester thereof, for manufacturing a pharmaceutical composition or a formulation; wherein the pharmaceutical composition or formulation is used for preventing and/or treating an artery disease selected from the group consisting of: (i) aneurysm; (ii) intramural hematoma; and (iii) arterial dissection; 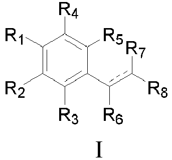
wherein, R₁, R₂, R₃, R₄ and R₅ are each independently selected from the group consisting of H, halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10-membered heteroaryl having 1-3 heteroatoms selected from N, S and O;
R₆ and R₇ are each independently selected from the group consisting of H, oxo (=O), halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
each R₁₀ is independently selected from the group consisting of -(C=O)-substituted or unsubstituted C1-C8 alkyl, -(C=O)-substituted or unsubstituted C6-C10 aryl, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₇ together with an atom to which they are attached form substituted or unsubstituted C3-C8 cycloalkyl, or substituted or unsubstituted 3-8 membered heterocycloalkyl having 1-3 heteroatoms selected from N, S and O;
" 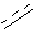 " is a double bond or a single bond;
R₈ is selected from the group consisting of H, -(C=O)-R₉, substituted or unsubstituted C1-C8 alkyl, substituted or unsubstituted C2-C8 alkenyl, substituted or unsubstituted C2-C8 alkynyl, and =CRₐR_{b};
R₉ is selected from the group consisting of H, hydroxy, substituted or unsubstituted C1-C8 alkyl, substituted or unsubstituted C2-C8 alkenyl, substituted or unsubstituted C2-C8 alkynyl, -NRₐR_{b}, and -O-R₁₁;
R₁₁ is selected from the group consisting of substituted or unsubstituted C1-C8 alkyl, substituted or unsubstituted C2-C8 alkenyl, substituted or unsubstituted C2-C8 alkynyl, -substituted or unsubstituted C1-C8 alkylene-C6-C10 aryl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₉ form 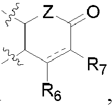 wherein Z is O, NRₐ or S;
unless especially indicated, the "substituted" refers to be substituted with one or more (e.g., 2, 3 or 4) substituents selected from the group consisting of halogen, deuterium, C1-C6 alkyl, C2-C6 alkenyl, C2-C6 alkynyl, C1-C6 alkoxy, halogenated C1-C6 alkyl, halogenated C1-C6 alkoxy, oxo (=O), -CN, -OH, -N(Rₐ)R_{b}, carboxyl, C1-C6 ester group (-C(=O)-OC1-C5 alkyl or -O-C(=O)C1-C5 alkyl), or substituted or unsubstituted group selected from the group consisting of: C1-C6 alkyl, C3-C8 cycloalkyl, C1-C6 amine group, C1-C6 ester group, C6-C10 aryl, 5-10 membered heteroaryl having 1-3 heteroatoms selected from N, S and O, 5-10 membered heterocyclyl having 1-3 heteroatoms selected from N, S and O, -(CH₂)-C6-C10 aryl, -(CH₂)-(5-10 membered heteroaryl having 1-3 heteroatoms selected from N, S and O), and the substituent is selected from the group consisting of: halogen, C1-C6 alkyl, C1-C6 alkynyl, C1-C6 alkoxy, oxo, -CN, -NH₂, -OH, C6-C10 aryl, C1-C6 amine group, C2-C6 amide group, and 5-10 heteroaryl having 1-3 heteroatoms selected from N, S and O;
each of Rₐ and R_{b} is independently selected from the group consisting of H, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O.

2. The use according to claim 1, wherein the use is for manufacturing a pharmaceutical composition or formulation; wherein the pharmaceutical composition or formulation is used for prevention and/or treatment of an arteriopathy selected from: (i) aneurysm; (ii) intramural hematoma; and/or (iii) arterial dissection; 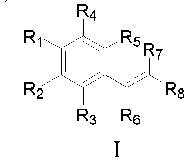
wherein, R₁, R₂, R₃, R₄, R₅, R₆ and R₇ are each independently selected from the group consisting of H, halogen, CN, OH, -O-R₁₀, -NRₐR_{b}, substituted or unsubstituted C1-C8 alkyl, substituted or unsubstituted C2-C8 alkenyl, substituted or unsubstituted C2-C8 alkynyl, -(C=O)-substituted or unsubstituted C1-C8 alkyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
R₁₀ is selected from the group consisting of -(C=O)-substituted or unsubstituted C1-C8 alkyl, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₇ together with the atom to which they are attached form substituted or unsubstituted C3-C8 cycloalkyl, or substituted or unsubstituted 3-8 membered heterocycloalkyl having 1-3 heteroatoms selected from N, S and O;
" 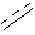 " is a double bond or a single bond;
Rs is selected from the group consisting of -(C=O)-R₉, substituted or unsubstituted C1-C8 alkyl, substituted or unsubstituted C2-C8 alkenyl, substituted or unsubstituted C2-C8 alkynyl, and =CRₐR_{b};
R₉ is selected from the group consisting of H, hydroxy, substituted or unsubstituted C1-C8 alkyl, substituted or unsubstituted C2-C8 alkenyl, substituted or unsubstituted C2-C8 alkynyl, -NRₐR_{b}, and -O-R₁₁;
R₁₁ is selected from the group consisting of substituted or unsubstituted C1-C8 alkyl, substituted or unsubstituted C2-C8 alkenyl, substituted or unsubstituted C2-C8 alkynyl, -substituted or unsubstituted C1-C8 alkylene-C6-C10 aryl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O;
or R₅ and R₉ form 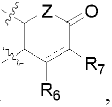 wherein Z is O, NRₐ or S;
unless especially indicated, the "substituted" refers to be substituted with one or more (e.g., 2, 3 or 4) substituents selected from the group consisting of halogen, deuterium, C1-C6 alkyl, C2-C6 alkenyl, C2-C6 alkynyl, C1-C6 alkoxy, halogenated C1-C6 alkyl, halogenated C1-C6 alkoxy, oxo (=O), -CN, -OH, -N(Rₐ)R_{b}, carboxyl, C1-C6 ester group (-C(=O)-OC1-C5 alkyl or -O-C(=O)C1-C5 alkyl), or substituted or unsubstituted group selected from the group consisting of: C1-C6 alkyl, C3-C8 cycloalkyl, C1-C6 amine group, C1-C6 ester group, C6-C10 aryl, 5-10 membered heteroaryl having 1-3 heteroatoms selected from N, S and O, 5-10 membered heterocyclyl having 1-3 heteroatoms selected from N, S and O, -(CH₂)-C6-C10 aryl, -(CH₂)-(5-10 membered heteroaryl having 1-3 heteroatoms selected from N, S and O, and the substituent is selected from the group consisting of: halogen, C1-C6 alkyl, C1-C6 alkynyl, C1-C6 alkoxy, oxo, -CN, -NH₂, -OH, C6-C10 aryl, C1-C6 amine group, C2-C6 amide group, and 5-10 heteroaryl having 1-3 heteroatoms selected from N, S and O;
each of Rₐ and R_{b} is independently selected from H, substituted or unsubstituted C1-C8 alkyl, substituted or unsubstituted C2-C8 alkenyl, substituted or unsubstituted C2-C8 alkynyl, substituted or unsubstituted C3-C10 cycloalkyl, substituted or unsubstituted 3-10 membered heterocycloalkyl having 1-3 heteroatoms selected from N, S and O, substituted or unsubstituted C6-C10 aryl, and substituted or unsubstituted 5-10 membered heteroaryl having 1-3 heteroatoms selected from N, S and O.

3. The use according to claim 1, wherein the " 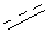 " is a double bond.

4. The use according to claim 1, wherein at least one of R₁, R₂, R₃, R₄ and R₅ is hydroxy or C1-C8 alkoxy.

5. The use according to claim 1, wherein the compound of formula I has a structure of formula Ia: 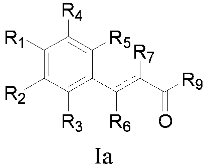 wherein, R₁, R₂, R₃, R₄, R₅, R₆, R₇, R₉ and " 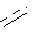 " are as defined in claim 1.

6. The use according to claim 5, wherein both R₁ and R₉ are OH.

7. The use according to claim 1, wherein the compound has a structure of formula Ib: 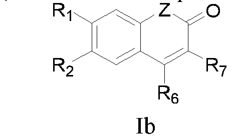 wherein, R₁, R₂, R₆, R₇ and Z are as defined in claim 1.

8. The use according to claim 1, wherein the compound has a structure of formula Ic: 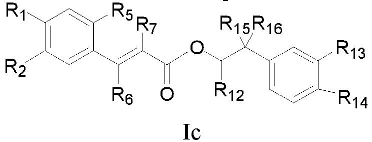
wherein, R₁, R₂, R₅, R₆ and R₇ are as defined in claim 1,
R₁₂ is selected from the group consisting of: H, halogen, -OH, substituted carboxyl or unsubstituted carboxyl, -C2-C8 ester group, substituted or unsubstituted C1-C6 alkyl, substituted or unsubstituted C1-C6 alkoxy;
R₁₃ and R₁₄ are each independently selected from the group consisting of H, -OH, halogen, C1-C4 alkyl, C1-C4 haloalkyl, and C1-C6 alkoxy; and
R₁₅ and R₁₆ are each independently selected from the group consisting of H, halogen, -OH, carboxyl, substituted or unsubstituted C1-C6 alkyl, and substituted or unsubstituted C1-C6 alkoxy.

9. The use according to claim 1, wherein the compound is selected from the following group A:

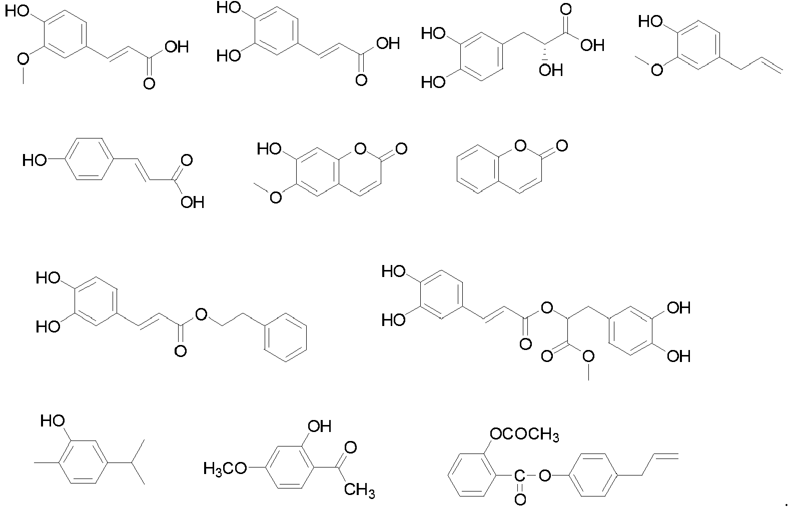

10. The use according to claim 1, wherein the artery is selected from the group consisting of thoracic aorta, abdominal aorta, splenic artery, hepatic artery, superior mesenteric artery, coeliac axis artery, renal artery, epiploon artery, inferior mesenteric artery, intracranial artery, carotid artery and a combination thereof.

11. The use according to claim 1, wherein the aneurysm is selected from the group consisting of early aneurysm, mid-term aneurysm, late aneurysm and a combination thereof.

12. A use of a medicinal material or food material containing a compound of formula I and/or an extract containing a compound of formula I, for manufacturing a composition; wherein the composition is used for prevention and/or treatment of an arterial disease selected from the group consisting of: (i) aneurysm; (ii) intramural hematoma; and (iii) arterial dissection; 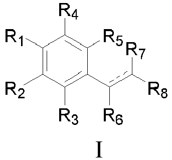
wherein, R₁, R₂, R₃, R₄, R₅, R₆ and R₇ and " 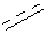 " are as defined in claim 1;
and, the compound of formula I is selected from: ferulic acid, caffeic acid, Danshensu, phenethyl caffeinate, methyl rosmarinate, coumarin, *p*-coumarin acid, scopoletin, eugenol, carvacrol, paeonol, aspirin eugenol, and a combination thereof.

13. The use according to claim 12, wherein the medicinal material is selected from: propolis, peppermint, *Ferulae Resina, Angelicae Sinensis Radix, Selaginellae Herba, Equiseti Hiemalis Herba, Chuanxiong Rhizoma, Cimicifugae Rhizoma, Ziziphi Spinosae Semen, Flos Anisopappi Chinensis, Caryophylli Flos, Stachys Palustris L., Fagopyri Dibotryis Rhizoma, Elaeagnus Multiflora Thunb, Cnidii Fructus, Rabdosiae Rubescentis Herba, Vitex Negundo L., South Salviae Miltiorrhizae Radix Et Rhizoma, Rabdosia Serra, Fraxini Cortex, Artemisiae Scopariae Herba, Angelicae Pubescentis Radix, Daphne odora Thunb. Hedyotis diffusa, Ginseng Radix Et Rhizoma, Cuscutae Semen, leaf of Juglans regia L., Murrayae Folium Et Cacumen, Cinnamomum Purpureum, Alpinia Galanga (L.) Willd, Magnoliae Flos, Narcissus Tazetta L., Commiphora Myrrha Engl., Rosae Rugosae Flos, Thymus Mongolicus Ronn, Cestrum Nocturnum L.,* and a combination thereof.

14. A pharmaceutical composition comprising:
(a) a first active ingredient selected from the group consisting of: a compound of formula I, an isomer thereof, a crystal form thereof, a hydrate or solvate thereof, or a pharmaceutically acceptable salt thereof, and a combination thereof; wherein the compound of formula I is as described in claim 1;
(b) a second active ingredient selected from the group consisting of: polymeric salvianolic acid, a stereoisomer thereof, a crystal form thereof, a pharmaceutically acceptable salt thereof, and a combination thereof; and
(c) a pharmaceutically acceptable carrier.

15. A method of preventing and/or treating: (i) aneurysm; (ii) arterial intramural hematoma; and/or (iii) arterial dissection, comprising a step of: administering a therapeutically effective amount of the compound of formula I according to claim 1, an isomer thereof, a crystal form thereof, a hydrate or a solvate thereof, or a pharmaceutically acceptable salt thereof, or a pharmaceutical composition containing them.
